# EUROPEAN PATENT SPECIFICATION

(11) **EP 4 058 438 B1**
(45) Date of publication and mention of the grant of the patent: **31.01.2024**
(21) Application number: 20808037.4
(22) Date of filing: 13.11.2020
(51) Int. Cl.: C07D 257/04, C07D 271/04, A01N 43/713, A01N 43/82

(54) **HERBICIDAL COMPOUNDS**
HERBIZIDE VERBINDUNGEN
COMPOSÉS HERBICIDES

(30) Priority: 15.11.2019 IN 201911046699
(43) Date of publication of application: 21.09.2022
(73) Proprietor: Syngenta Crop Protection AG, 4058 Basel (CH)
(72) Inventor: BURTON, Paul, Matthew, Bracknell Berkshire RG42 6EY (GB); RAJAN, Ramya, Corlim IIhas Goa 403 110 (IN); EMERY, Katie, Bracknell Berkshire RG42 6EY (GB); MITCHELL, Glynn, Bracknell Berkshire RG42 6EY (GB); BURNS, David, Bracknell Berkshire RG42 6EY (GB); MCGRANAGHAN, Andrea, Bracknell Berkshire RG42 6EY (GB)
(74) Representative: SYNGENTA IP
(86) International application number: PCT/EP2020/081995
(87) International publication number: WO 2021/094505

(56) References cited:
- WO-A1-2012/028579

## Description

The present invention relates to novel herbicidal compounds, to processes for their preparation, to herbicidal compositions which comprise the novel compounds, and to their use for controlling weeds, in particular in crops of useful plants, or for inhibiting plant growth.

N-(tetrazol-5-yl)- and N-(1,3,4-oxadiazol-2-yl) arylcarboxamides are disclosed in, for example, WO2012/028579 and WO2012/126932 respectively. The present invention relates to novel arylcarboxamides.

Thus, according to the present invention there is provided a compound of Formula (I): 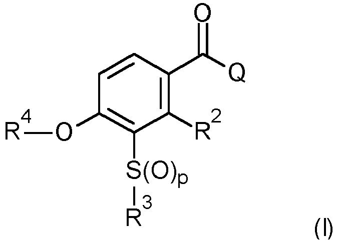 or an agronomically acceptable salt thereof,
wherein
Q is selected from the group consisting of Q1 and Q2: 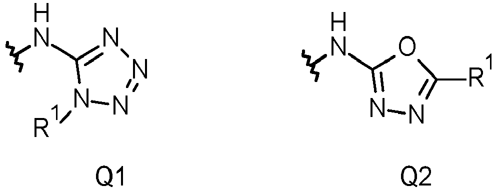
R¹ is selected from the group consisting of C₁-C₄alkyl-, C₁-C₄haloalkyl-, C₁-C₄alkoxy-C₁-C₄alkyl- and C₁-C₄haloalkoxy-C₁-C₄alkyl-;
R² is selected from the group consisting of halogen, C₁-C₆alkyl-, C₁-C₃alkoxy-, C₁-C₆ haloalkyl-, C₁-C₃haloalkoxy- and -S(O)ₚC₁-C₆alkyl;
R³ is selected from the group consisting of C₁-C₆alkyl-, C₃-C₆cycloalkyl- and C₁-C₆ haloalkyl-;
R⁴ is C₁-C₆haloalkyl;
   and
p is 0, 1 or 2.

C₁-C₆alkyl and C₁-C₄alkyl groups include, for example, methyl (Me, CH₃), ethyl (Et, C₂H₅), *n*-propyl (*n*-Pr), isopropyl (*i*-Pr), *n*-butyl (*n*-Bu), isobutyl (*i*-Bu), *sec*-butyl and *tert-*butyl (*t*-Bu).

Halogen (or halo) encompasses fluorine, chlorine, bromine or iodine. The same correspondingly applies to halogen in the context of other definitions, such as haloalkyl.

C₁-C₆alkoxyC₁-C₃alkyl- includes, for example, methoxyethyl- and ethoxymethyl-.

C₃-C₆ cycloalkyl as used herein refers to a stable, monocyclic ring radical which is saturated and contains 3 to 6 carbon atoms. Examples of C₃₋₆cycloalkyl include cyclopropyl, cyclobutyl, cyclopentyl and cyclohexyl.

C₁-C₆haloalkyl includes, for example, fluoromethyl-, difluoromethyl-, trifluoromethyl-, chloromethyl-, dichloromethyl-, trichloromethyl-, 2,2,2-trifluoroethyl-, 2-fluoroethyl-, 2-chloroethyl-, pentafluoroethyl-, 1,1-difluoro-2,2,2-trichloroethyl-, 2,2,3,3-tetrafluoroethyl-, 2,2,2-trichloroethyl-, heptafluoro-n-propyl and perfluoro-n-hexyl. C₁-C₄haloalkyl includes, for example, fluoromethyl-, difluoromethyl-, trifluoromethyl-, chloromethyl-, dichloromethyl-, trichloromethyl-, 2,2,2-trifluoroethyl-, 2-fluoroethyl-, 2-chloroethyl-, pentafluoroethyl-, 1,1-difluoro-2,2,2-trichloroethyl-, 2,2,3,3-tetrafluoroethyl-, 2,2,2-trichloroethyl- and heptafluoro-n-propyl-.

C₁-C₆alkyl-S- (alkylthio) is, for example, methylthio, ethylthio, propylthio, isopropylthio, *n*-butylthio, isobutylthio, sec-butylthio or tert-butylthio, preferably methylthio or ethylthio.

C₁-C₆alkyl-S(O)- (alkylsulfinyl) is, for example, methylsulfinyl, ethylsulfinyl, propylsulfinyl, isopropylsulfinyl, n-butylsulfinyl, isobutylsulfinyl, sec-butylsulfinyl or tert-butylsulfinyl, preferably methylsulfinyl or ethylsulfinyl.

C₁-C₆alkyl-S(O)₂- (alkylsulfonyl) is, for example, methylsulfonyl, ethylsulfonyl, propylsulfonyl, isopropylsulfonyl, n-butylsulfonyl, isobutylsulfonyl, sec-butylsulfonyl or tert-butylsulfonyl, preferably methylsulfonyl or ethylsulfonyl.

In one embodiment of the present invention there is provided a compound of Formula (I) wherein Q is Q1 (shown below as a Compound of Formula (Ia)). In another embodiment of the present invention there is provided a compound of Formula (I) wherein Q is Q2 (shown below as a Compound of Formula (Ib)).

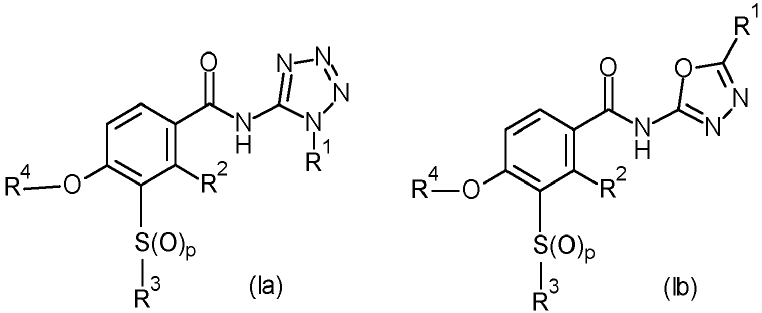

In a preferred embodiment of the present invention R¹ is selected from the group consisting of C₁-C₄alkyl- (preferably methyl, ethyl or *n*-propyl), C₁-C₄haloalkyl- (preferably 2,2-difluoroethyl or 2,2,2-trifluoroethyl) and C₁-C₄alkoxy-C₁-C₄alkyl- (preferably methoxyethyl-). In a more preferred embodiment R¹ is selected from the group consisting of methyl, ethyl and *n*-propyl. In an especially preferred embodiment of the present invention R¹ is methyl.

In one embodiment of the present invention, R² is selected from the group consisting of halogen (preferably chlorine), C₁-C₆alkyl- (preferably methyl), C₁-C₃alkoxy- (preferably methoxy-), C₁-C₆ haloalkyl- (preferably -CF₃), C₁-C₃haloalkoxy- (preferably CF₃O-) and - S(O)ₚC₁-C₆alkyl (preferably -SO₂Me). In an especially preferred embodiment, R² is chlorine.

In one embodiment of the present invention, R³ is selected from the group consisting of C₁-C₆alkyl- (preferably methyl or ethyl), C₃-C₆cycloalkyl (e.g cyclopropyl) and C₁-C₆haloalkyl- (e.g -CF₃). In a preferred embodiment of the present invention, R³ is methyl or ethyl, most preferably methyl.

In a preferred embodiment of the present invention R⁴ is selected from the group consisting of CF₃-, CHF₂-, CH₃CF₂-, CF₃CH₂-, CF₂HCF₂- and CF₃CHFCF₂-. In a more preferred embodiment, R⁴ is CF₃- or CHF₂-, most preferably CF₃-.

The present invention also includes agronomically acceptable salts that the compounds of Formula (I) may form with amines (for example ammonia, dimethylamine and triethylamine), alkali metal and alkaline earth metal bases or quaternary ammonium bases. Among the alkali metal and alkaline earth metal hydroxides, oxides, alkoxides and hydrogen carbonates and carbonates used as salt formers, emphasis is to be given to the hydroxides, alkoxides, oxides and carbonates of lithium, sodium, potassium, magnesium and calcium, but especially those of sodium, magnesium and calcium. The corresponding trimethylsulfonium salt may also be used.

The compounds of Formula (I) according to the invention can be used as herbicides by themselves, but they are generally formulated into herbicidal compositions using formulation adjuvants, such as carriers, solvents and surface-active agents (SFAs). Thus, the present invention further provides a herbicidal composition comprising a herbicidal compound of the present invention and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

The herbicidal compositions generally comprise from 0.1 to 99 % by weight, especially from 0.1 to 95 % by weight, compounds of Formula I and from 1 to 99.9 % by weight of a formulation adjuvant which preferably includes from 0 to 25 % by weight of a surface-active substance.

The compositions can be chosen from a number of formulation types, many of which are known from the Manual on Development and Use of FAO Specifications for Plant Protection Products, 5th Edition, 1999. These include dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, capsule suspensions (CS) and seed treatment formulations. The formulation type chosen in any instance will depend upon the particular purpose envisaged and the physical, chemical and biological properties of the compound of Formula (I).

Dustable powders (DP) may be prepared by mixing a compound of Formula (I) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from pre-formed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of Formula (I) is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example n-butane). A compound of Formula (I) may also be dissolved or dispersed in a suitable medium (for example water or a water miscible liquid, such as n-propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SFAs), spray additives based on oils, for example certain mineral oils or natural plant oils (such as soy bean and rape seed oil), and blends of these with other bio-enhancing adjuvants (ingredients which may aid or modify the action of a compound of Formula (I).

Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium diisopropyl- and tri-isopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately diesters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

The herbicidal compounds of present invention can also be used in mixture with one or more additional herbicides and/or plant growth regulators. Examples of such additional herbicides or plant growth regulators include acetochlor, acifluorfen (including acifluorfen-sodium), aclonifen, ametryn, amicarbazone, aminopyralid, aminotriazole, atrazine, beflubutamid-M, bensulfuron (including bensulfuron-methyl), bentazone, bicyclopyrone, bilanafos, bispyribac-sodium, bixlozone, bromacil, bromoxynil, butachlor, butafenacil, carfentrazone (including carfentrazone-ethyl), cloransulam (including cloransulam-methyl), chlorimuron (including chlorimuron-ethyl), chlorotoluron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, diquat dibromide, diuron, ethalfluralin, ethofumesate, fenoxaprop (including fenoxaprop-P-ethyl), fenoxasulfone, fenquinotrione, fentrazamide, flazasulfuron, florasulam, florpyrauxifen (including florpyrauxifen-benzyl), fluazifop (including fluazifop-P-butyl), flucarbazone (including flucarbazone-sodium), flufenacet, flumetsulam, flumioxazin, fluometuron, flupyrsulfuron (including flupyrsulfuron-methyl-sodium), fluroxypyr (including fluroxypyr-meptyl), fomesafen, foramsulfuron, glufosinate (including the ammonium salt thereof), glyphosate (including the diammonium, isopropylammonium and potassium salts thereof), halauxifen (including halauxifen-methyl), haloxyfop (including haloxyfop-methyl), hexazinone, hydantocidin, imazamox, imazapic, imazapyr, imazethapyr, indaziflam, iodosulfuron (including iodosulfuron-methyl-sodium), iofensulfuron (including iofensulfuron-sodium), ioxynil, isoproturon, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, prometryne, propanil, propaquizafop, propyrisulfuron, propyzamide, prosulfocarb, prosulfuron, pyraclonil, pyraflufen (including pyraflufen-ethyl), pyrasulfotole, pyridate, pyriftalid, pyrimisulfan, pyroxasulfone, pyroxsulam, quinclorac, quinmerac, quizalofop (including quizalofop-P-ethyl and quizalofop-P-tefuryl), rimsulfuron, saflufenacil, sethoxydim, simazine, S-metalochlor, sulfentrazone, sulfosulfuron, tebuthiuron, tefuryltrione, tembotrione, terbuthylazine, terbutryn, tetflupyrolimet, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, ethyl 2-[[3-[2-chloro-4-fluoro-5-[3-methyl-2,6-dioxo-4-(trifluoromethyl)pyrimidin-1-yl]phenoxy]-2-pyridyl]oxy]acetate, 3-(2-chloro-4-fluoro-5-(3-methyl-2,6-dioxo-4-trifluoromethyl-3,6-dihydropyrimidin-1(2H)-yl)phenyl)-5-methyl-4,5-dihydroisoxazole-5-carboxylic acid ethyl ester, 4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, 3-[2-(3,4-dimethoxyphenyl)-6-methyl-3-oxo-pyridazine-4-carbonyl]bicyclo[3.2.1]octane-2,4-dione, 2-[2-(3,4-dimethoxyphenyl)-6-methyl-3-oxo-pyridazine-4-carbonyl]-5-methyl-cyclohexane-1,3-dione, 2-[2-(3,4-dimethoxyphenyl)-6-methyl-3-oxo-pyridazine-4-carbonyl]cyclohexane-1,3-dione, 2-[2-(3,4-dimethoxyphenyl)-6-methyl-3-oxo-pyridazine-4-carbonyl]-5,5-dimethyl-cyclohexane-1,3-dione, 6-[2-(3,4-dimethoxyphenyl)-6-methyl-3-oxo-pyridazine-4-carbonyl]-2,2,4,4-tetramethyl-cyclohexane-1,3,5-trione, 2-[2-(3,4-dimethoxyphenyl)-6-methyl-3-oxo-pyridazine-4-carbonyl]-5-ethyl-cyclohexane-1,3-dione, 2-[2-(3,4-dimethoxyphenyl)-6-methyl-3-oxo-pyridazine-4-carbonyl]-4,4,6,6-tetramethyl-cyclohexane-1,3-dione, 2-[6-cyclopropyl-2-(3,4-dimethoxyphenyl)-3-oxo-pyridazine-4-carbonyl]-5-methyl-cyclohexane-1,3-dione, 3-[6-cyclopropyl-2-(3,4-dimethoxyphenyl)-3-oxo-pyridazine-4-carbonyl]bicyclo[3.2.1]octane-2,4-dione, 2-[6-cyclopropyl-2-(3,4-dimethoxyphenyl)-3-oxo-pyridazine-4-carbonyl]-5,5-dimethylcyclohexane-1,3-dione, 6-[6-cyclopropyl-2-(3,4-dimethoxyphenyl)-3-oxo-pyridazine-4-carbonyl]-2,2,4,4-tetramethyl-cyclohexane-1,3,5-trione, 2-[6-cyclopropyl-2-(3,4-dimethoxyphenyl)-3-oxo-pyridazine-4-carbonyl]cyclohexane-1,3-dione, 4-[2-(3,4-dimethoxyphenyl)-6-methyl-3-oxo-pyridazine-4-carbonyl]-2,2,6,6-tetramethyl-tetrahydropyran-3,5-dione, 4-[6-cyclopropyl-2-(3,4-dimethoxyphenyl)-3-oxo-pyridazine-4-carbonyl]-2,2,6,6-tetramethyl-tetrahydropyran-3,5-dione and 4-amino-3-chloro-5-fluoro-6-(7-fluoro-1H-indol-6-yl)pyridine-2-carboxylic acid (including agrochemically acceptable esters thereof, for example, methyl 4-amino-3-chloro-5-fluoro-6-(7-fluoro-1H-indol-6-yl)pyridine-2-carboxylate).

The mixing partners of the compound of Formula (I) may also be in the form of esters or salts, as mentioned e.g. in The Pesticide Manual, Sixteenth Edition, British Crop Protection Council, 2012.

The compound of Formula (I) can also be used in mixtures with other agrochemicals such as fungicides, nematicides or insecticides, examples of which are given in The Pesticide Manual.

The mixing ratio of the compound of Formula (I) to the mixing partner is preferably from 1: 100 to 1000:1.

The mixtures can advantageously be used in the above-mentioned formulations (in which case "active ingredient" relates to the respective mixture of compound of Formula (I) with the mixing partner).

The compounds or mixtures of the present invention can also be used in combination with one or more herbicide safeners. Examples of such safeners include benoxacor, cloquintocet (including cloquintocet-mexyl), cyprosulfamide, dichlormid, fenchlorazole (including fenchlorazole-ethyl), fenclorim, fluxofenim, furilazole, isoxadifen (including isoxadifen-ethyl), mefenpyr (including mefenpyr-diethyl), metcamifen and oxabetrinil.

Particularly preferred are mixtures of a compound of Formula (I) with cyprosulfamide, isoxadifen-ethyl, cloquintocet-mexyl and/or metcamifen.

The safeners of the compound of Formula (I) may also be in the form of esters or salts, as mentioned e.g. in The Pesticide Manual, 16th Edition (BCPC), 2012. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048.

Preferably the mixing ratio of compound of Formula (I) to safener is from 100:1 to 1:10, especially from 20:1 to 1:1.

The present invention still further provides a method of controlling weeds at a locus said method comprising application to the locus of a weed controlling amount of a composition comprising a compound of Formula (I). Moreover, the present invention further provides a method of selectively controlling weeds at a locus comprising crop plants and weeds, wherein the method comprises application to the locus of a weed controlling amount of a composition according to the present invention. 'Controlling' means killing, reducing or retarding growth or preventing or reducing germination. Generally the plants to be controlled are unwanted plants (weeds). 'Locus' means the area in which the plants are growing or will grow. Some crop plants may be inherently tolerant to herbicidal effects of compounds of Formula (I). However, in some instances tolerance may need to be engineered into the crop plant, for example by way of genetic engineering. Thus, it is possible that the crop plant is rendered tolerant to HPPD-inhibitors via genetic engineering. Methods of rending crop plants tolerant to HPPD-inhibitors are known, for example from WO0246387. Thus in an even more preferred embodiment the crop plant is transgenic in respect of a polynucleotide comprising a DNA sequence which encodes an HPPD-inhibitor resistant HPPD enzyme derived from a bacterium, more particularly from *Pseudomonas fluorescens* or *Shewanella colwelliana*, or from a plant, more particularly, derived from a monocot plant or, yet more particularly, from a barley, maize, wheat, rice, *Brachiaria, Cenchrus, Lolium, Festuca, Setaria, Eleusine, Sorghum* or *Avena* species. Several HPPD-tolerant soybean transgenic "events" are known, and include for example SYHT04R (WO2012/082542), SYHT0H2 (WO2012/082548) and FG72. Other polynucleotide sequences that can be used to provide plants which are tolerant to the compounds of the present invention are disclosed in, for example, WO2010/085705 and WO2011/068567. Crop plants in which the composition according to the invention can be used thus include crops such as cereals, for example barley and wheat, cotton, oilseed rape, sunflower, maize, rice, soybeans, sugar beet, sugar cane and turf.

Crop plants can also include trees, such as fruit trees, palm trees, coconut trees or other nuts. Also included are vines such as grapes, fruit bushes, fruit plants and vegetables.

The rates of application of compounds of Formula I may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula I according to the invention are generally applied at a rate of from 10 to 2000 g/ha, especially from 50 to 1000 g/ha.

The application is generally made by spraying the composition, typically by tractor mounted sprayer for large areas, but other methods such as dusting (for powders), drip or drench can also be used.

Crop plants are to be understood as also including those crop plants which have been rendered tolerant to herbicides or classes of herbicides (e.g. ALS-, GS-, EPSPS-, PPO-, ACCase- and HPPD-inhibitors) by conventional methods of breeding or by genetic engineering. An example of a crop that has been rendered tolerant to imidazolinones, e.g. imazamox, by conventional methods of breeding is Clearfield^{®} summer rape (canola). Examples of crops that have been rendered tolerant to herbicides by genetic engineering methods include e.g. glyphosate- and glufosinate-resistant maize varieties commercially available under the trade names RoundupReady^{®} and LibertyLink^{®}.

Crop plants are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK^{®} (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut^{®} (maize), Yield Gard^{®} (maize), NuCOTIN33B^{®} (cotton), Bollgard^{®} (cotton), NewLeaf^{®} (potatoes), NatureGard^{®} and Protexcta^{®}. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crop plants are also to be understood to include those which are obtained by conventional methods of breeding or genetic engineering and contain so-called output traits (e.g. improved storage stability, higher nutritional value and improved flavour).

Other useful plants include turf grass for example in golf-courses, lawns, parks and roadsides, or grown commercially for sod, and ornamental plants such as flowers or bushes.

The compositions can be used to control unwanted plants (collectively, 'weeds'). The weeds to be controlled may be both monocotyledonous species, for example Agrostis, Alopecurus, Avena, Brachiaria, Bromus, Cenchrus, Cyperus, Digitaria, Echinochloa, Eleusine, Lolium, Monochoria, Rottboellia, Sagittaria, Scirpus, Setaria and Sorghum, and dicotyledonous species, for example Abutilon, Amaranthus, Ambrosia, Chenopodium, Chrysanthemum, Conyza, Galium, Ipomoea, Nasturtium, Sida, Sinapis, Solanum, Stellaria, Veronica, Viola and Xanthium. Weeds can also include plants which may be considered crop plants but which are growing outside a crop area ('escapes'), or which grow from seed left over from a previous planting of a different crop ('volunteers'). Such volunteers or escapes may be tolerant to certain other herbicides.

The present invention further provides a compound of Formula (Va) 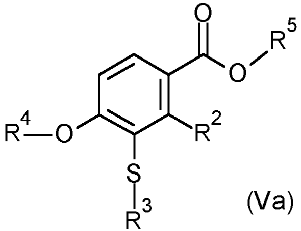 wherein R², R³ and R⁴ are as defined in claim 1 above and R⁵ is hydrogen or C₁-C₄ alkyl.

The compounds of the present invention can be prepared according to the following schemes. Compounds of formula (I) where p=2 or p=1 may be prepared from compounds of formula (I) where p=0.

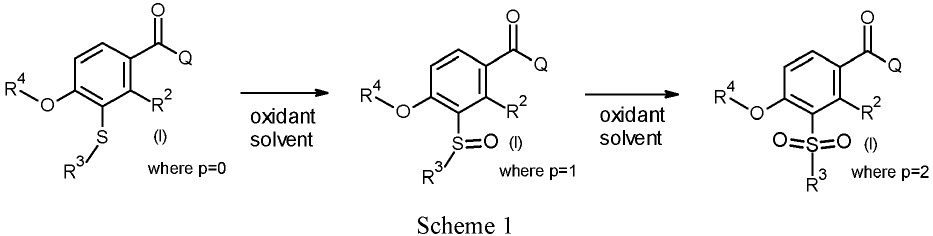

As shown in Scheme 1, the compound of formula (I) where p=0 is treated with a suitable oxidant (for example meta-chloroperoxybenzoic acid) in a suitable solvent (for example dichloromethane) to give the compound of formula (I) where p=1. The compound of formula (I) where p=1 may be further oxidised to the compound of formula (I) where p=2 by treatment with a suitable oxidant (for example meta-chloroperoxybenzoic acid) in a suitable solvent (for example dichloromethane). The skilled person will recognise that the compound of formula (I) where p=2 may be prepared in one reaction from the compound of formula (I) where p=0 by treatment with at least 2 equivalents of the oxidant. The skilled person will recognise that the second oxidation, from the compound of formula (I) where p=1 to the compound of formula (II) where p=2, requires higher temperatures and longer reaction times compared to the first oxidation, from compound of formula (I) where p=0 to the compound of formula (I) where p=1. Therefore, the skilled person will be able to control the oxidation to give their desired compound of formula (I).

Amides of formula (I) where p=0 may be prepared from pentafluorophenyl esters of formula (II) and amines of formula (III) or formula (IV).

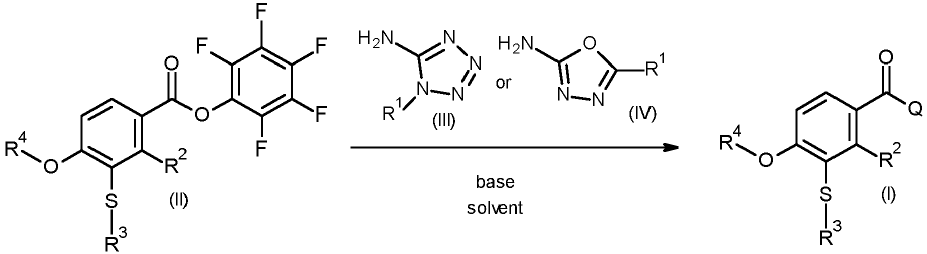

The pentafluorophenyl ester of formula (II) is treated with an amine of formula (III) (for Q=Q1) or an amine of formula (IV) (for Q=Q2) in the presence of a suitable base (for example 2-tert-butylimino-2-diethylamino-1,3-dimethylperhydro-1,3,2-diazaphosphorine) and in a suitable solvent (for example acetonitrile).

Pentafluorophenyl esters of formula (II) may be prepared from benzoic acids of formula (V).

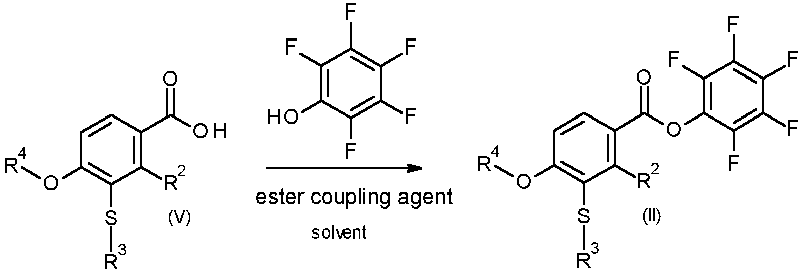

The benzoic acid of formula (V) is reacted with pentafluorophenol and a suitable ester coupling agent (for example 1-ethyl-3-(3-dimethylaminopropyl)carbodiimide) in a suitable solvent (for example dichloromethane).

In the embodiments of the invention where R² is C₁-C₆alkyl-, C₁-C₃alkoxy-, C₁-C₆ haloalkyl-, C₁-C₃haloalkoxy- and -S(O)ₚC₁-C₆alkyl, the benzoic acid of formula (V) may be prepared from an ester of formula (VI).

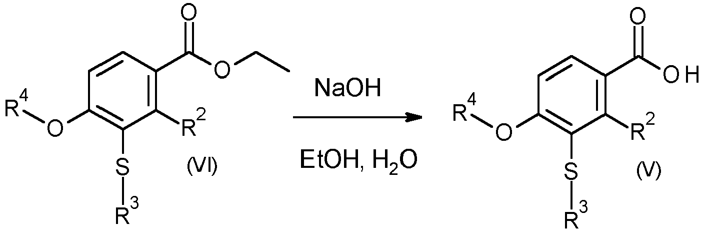

The ester of formula (VI) is treated with sodium hydroxide in an ethanol + water solvent to give the benzoic acid of formula (V).

In the embodiments of the invention where R² is C₁-C₆alkyl-, C₁-C₃alkoxy-, C₁-C₆ haloalkyl-, C₁-C₃haloalkoxy- or -S(O)ₚC₁-C⁶alkyl, the compound of formula (VI) may be prepared from compound of formula (VI) where R² is chloro.

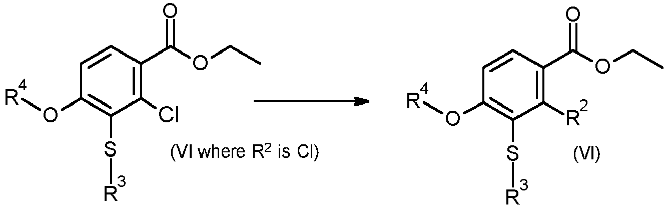

In this step, the chloro substituent is converted to the appropriate R² substituent of the compound of formula (VI). The method of this reaction will be dependent on the identity of R². The skilled person will be familiar with such transformations. For example, where R² is C1-C6alkyl, the compound of formula (IX) is reacted with a C₁-C₆alkyl boronic acid or C₁-C6alkyl boroxine (for example trimethylboroxine for R² = methyl) in the presence of a suitable catalyst (for example [1,3-bis(2,6-diisopropylphenyl)imidazol-2-ylidene](3-chloropyridyl)palladium(II) dichloride) and a suitable base (for example potassium carbonate) in a suitable solvent (for example 1,4-dioxane).

Compounds of formula (VI) where R² = chloro may be prepared from compounds of formula (V) where R² = chloro.

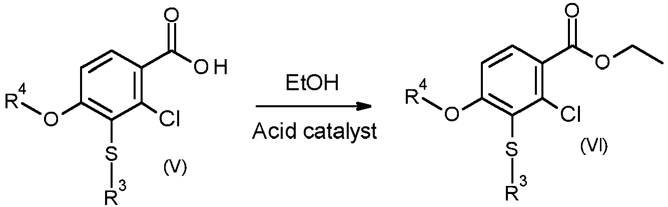

The compound of formula (V) is treated with ethanol and an acid catalyst (for example sulfuric acid) to give the compound of formula (VI).

Benzoic acids of formula (V) where R² = chloro may be prepared from compounds of formula (VII).

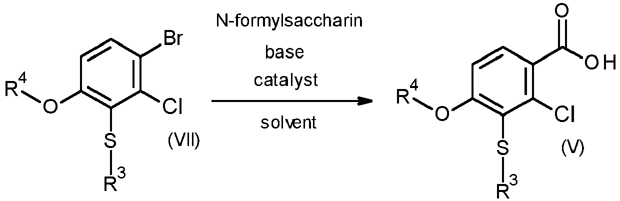

The compound of formula (VII) is treated with N-formylsaccharin and a suitable catalyst (for example palladium(II) acetate and Xantphos) and a suitable base (for example triethylamine) in a suitable solvent (for example N-methylpyrrolidinone and water).

Compounds of formula (VII) may be prepared from compounds of formula (VIII) and compounds of formula (IX).

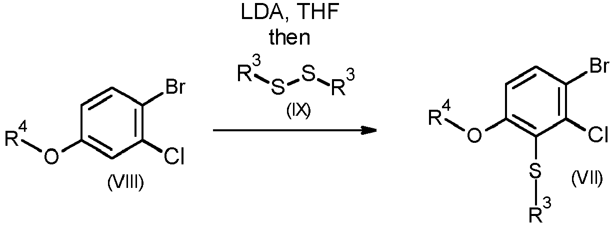

Compounds of formula (VIII) are treated with lithium diisopropylamide (LDA) in a suitable solvent (for example tetrahydrofuran), then a compound of formula (IX).

In an alternative method, compounds of formula (VII) may be prepared from phenols of formula (X).

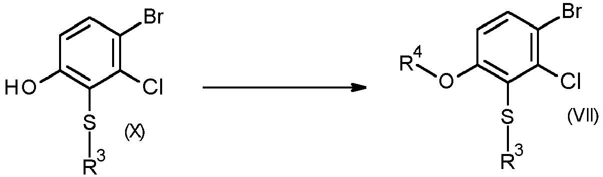

The phenol of formula X is treated with a suitable haloalkyation reagent, which will be different depending on the identity of R⁴. For example, where R⁴ is -CH₂CF₃, the phenol of formula X is treated with 2,2,2-triethyltrifluoromethylsulfonate and a base (for example potassium carbonate). In another example, where R4 is -CHF₂, the phenol of formula (X) is treated with sodium 2-chloro-2,2-difluoro-acetate and a base (for example potassium carbonate).

Phenols of formula (X) may be prepared from compounds of formula (XI).

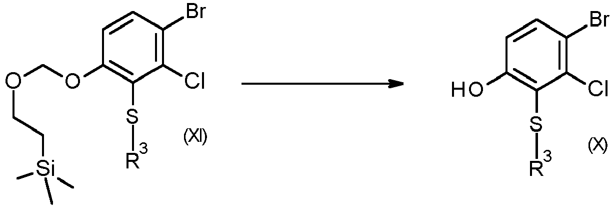

The compound of formula (XI) is treated with an aqueous acid (for example 2N hydrochloric acid) in methanol.

Compounds of formula (XI) may be prepared from (2-((4-bromo-3-chlorophenoxy)methoxy)ethyl)trimethylsilane.

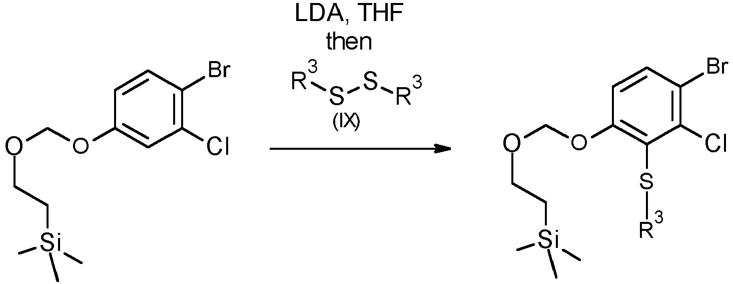

(2-((4-bromo-3-chlorophenoxy)methoxy)ethyl)trimethylsilane is treated with lithium diisopropylamide (LDA) in a solvent (for example tetrahydrofuran), then treated with a compound of formula (IX).

(2-((4-bromo-3-chlorophenoxy)methoxy)ethyl)trimethylsilane may be prepared from 4-bromo-3-chlorophenol.

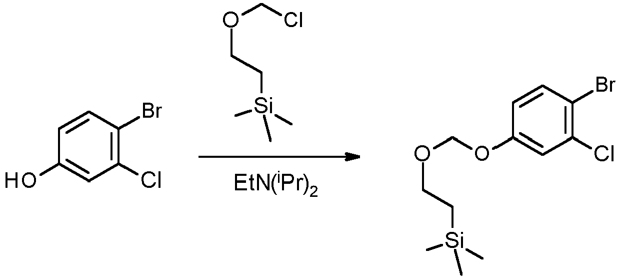

4-Bromo-3-chlorophenol is treated with 2-(chloromethoxy)ethyl-trimethyl-silane and N,N-diisopropylethylamine.

The following non-limiting examples provide specific synthesis methods for representative compounds of the present invention, as referred to the Tables provided herein.

### EXAMPLE 1. Preparation of Compound 1.001.

### Step 1

To a flask containing 4-bromo-3-chloro-phenol (8 g, 38.6 mmol) was added DCM (40 mL) and N,N-diisopropylethylamine (10 g, 13.5 mL, 77.1 mmol). At 0 °C, 2-(chloromethoxy)ethyl-trimethyl-silane (7.07 g, 7.4 mL, 42.4 mmol) was added dropwise. The reaction was stirred at room temperature overnight. The reaction was quenched by addition of water, then saturated aqueous NaHCOs, The material was extracted with ethyl acetate and the organic phase was concentrated *in vacuo* to give 2-[(4-bromo-3-chloro-phenoxy)methoxy]ethyl-trimethyl-silane as an orange oil (14.8 g, quant%) ¹H NMR (Chloroform): 7.47 (d,1H), 7.18 (d,1H), 6.83 (dd,1H), 5.18 (s,2H), 3.75 (m,2H), 0.94 (m,2H), 0.00 (m,9H)

### Step 2

To a 3 neck flask was added THF (280 mL) and the reaction mixture was purged and filled with N₂. Diisopropylamine (6.78 g, 9.44 mL, 66.3 mmol) was added. The reaction was stirred at -78 °C for 30 min. N-butyllithium in hexane (16 g, 2.5 mol/L, 23 mL, 58.0 mmol) was added dropwise *via* syringe pump (10 mL/min). This was stirred for 1 h, then the mixture was allowed to warm to -40 °C, then cooled to -78 °C again. A solution of 2-[(4-bromo-3-chloro-phenoxy)methoxy]ethyl-trimethyl-silane (14 g, 41.5 mmol) in 23 mL of THF was added *via* syringe pump (10 mL/min) and the reaction mixture was stirred at -78 °C for 3 h. Dimethyl disulfide (7.89 g, 7.54 mL, 82.9 mmol) in 16 mL THF was added dropwise (10 mL/min) and the mixture was stirred at -78 °C for 40 min. The reaction mixture was quenched by adding it cold into a stirred solution of water. 2M HCl was added until the mixture was acidic and the mixture was stirred for 15 min. The material was extracted with ethyl acetate and the organic phase was concentrated *in vacuo* to give 2-[(4-bromo-3-chloro-2-methylsulfanyl-phenoxy)methoxy]ethyl-trimethyl-silane (14.4 g, 31.1 mmol, 75%) as an orange oil. 1HNMR (Chloroform): 7.50 (d,1H), 7.01 (d,1H), 5.31 (s,2H), 3.79 (m,2H), 2.42 (s,3H), 0.95 (m,2H), 0.00 (s,9H)

### Step 3

To a flask containing 2-[(4-bromo-3-chloro-2-methylsulfanyl-phenoxy)methoxy]ethyl-trimethyl-silane (14.4 g, 37.5 mmol) was added THF (188 mL), MeOH (113 mL) and 2M aqueous HCl (113 mL). The reaction was stirred at 70 °C for 1 h. The reaction mixture was cooled to room temperature, then concentrated *in vacuo.* The crude material was diluted with water and extracted with ethyl acetate and the organic phase was concentrated *in vacuo.* The material was purified by flash chromatography (0 to 20% EtOAc in cyclohexane) to give 4-bromo-3-chloro-2-methylsulfanyl-phenol (8.28 g, 32.6 mmol, 87%) as white crystals. ¹H NMR (Chloroform): 7.50 (d,1H), 7.09 (s,1H), 6.85 (d,1H), 2.34 (s,3H)

### Step 4

To a flask containing 4-bromo-3-chloro-2-methylsulfanyl-phenol (2 g, 7.89 mmol) was added DMF (20 mL). K₂CO₃ (1.38 g, 9.47 mmol) was added, followed by 2,2,2-trifluoroethyl trifluoromethanesulfonate (2.20 g, 1.36 mL, 9.47 mmol) and the reaction mixture was stirred at room temperature for 3.5 h. The reaction mixture was quenched by addition of water and the material was extracted with ethyl acetate. The organic phase was washed with water and concentrated *in vacuo.* The material was purified by flash chromatography (0 to 15% EtOAc in cyclohexane) to give 1-bromo-2-chloro-3-methylsulfanyl-4-(2,2,2-trifluoroethoxy)benzene (2.62 g, 7.81 mmol, 99%) as a colourless oil. 1H NMR (Chloroform): 7.54 (d,1H), 6.73 (d,1H), 4.42 (q,2H), 2.45 (s,3H)

### Step 5

To a vessel containing NMP (101 mL) was added palladium(II) acetate (0.169 g, 0.751 mmol), XantPhos (0.896 g, 1.50 mmol), N-formylsaccharine (3.57 g, 16.9 mmol) and 1-bromo-2-chloro-3-methylsulfanyl-4-(2,2,2-trifluoroethoxy)benzene (2.52 g, 7.51 mmol). To a second vessel was added triethylamine (3.57 g, 4.71 mL, 33.8 mmol), NMP (101 mL) and water (5.04 mL). The reaction was carried out in a Uniqsis FlowSyn. The two solutions were pumped through a T-piece and then round a 20 mL stainless steel coil heated to 170 °C. The flow rate was set so that the total residence time was 15 min. The reaction mixture was cooled to room temperature and was diluted with ethyl acetate. The organic phase was washed with 2M HCl, then with water. The organic phase was concentrated *in vacuo.* The material was purified by flash chromatography (0 to 100% EtOAc in cyclohexane) to give 2-chloro-3-methylsulfanyl-4-(2,2,2-trifluoroethoxy)benzoic acid (0.96 g, 2.87 mmol, 38%) as an orange solid. 1H NMR (Methanol): 7.78 (d,1H), 7.09 (d,1H), 4.71 (q,2H), 2.40 (s,3H)

### Step 6

To a flask containing 2-chloro-3-methylsulfanyl-4-(2,2,2-trifluoroethoxy)benzoic acid (0.816 g, 2.71 mmol) was added 2,3,4,5,6-pentafluorophenol (0.750 g, 4.07 mmol) and DCM (16 mL). 1-(3-dimethylaminopropyl)-3-ethylcarbodiimide hydrochloride (0.602 g, 2.99 mmol) was added and the reaction was stirred at room temperature for 2 h. The reaction mixture was diluted with DCM and washed with saturated aqueous NaHCO₃. The organic phase was concentrated *in vacuo.* The material was purified by flash chromatography (0 to 10% EtOAc in cyclohexane) to give (2,3,4,5,6-pentafluorophenyl) 2-chloro-3-methylsulfanyl-4-(2,2,2-trifluoroethoxy)benzoate (0.758 g, 1.62 mmol, 60%) as white needles. 1H NMR (Chloroform): 8.07 (d,1H), 6.91 (d,1H), 4.54 (q,2H), 2.46 (s,3H)

### Step 7

To a flask containing the (2,3,4,5,6-pentafluorophenyl) 2-chloro-3-methylsulfanyl-4-(2,2,2-trifluoroethoxy)benzoate (0.379 g, 0.812 mmol) was added acetonitrile (7.6 mL) and the mixture was stirred at room temperature. 1-Methyltetrazol-5-amine (88.5 Mg, 0.893 mmol) was added, followed by 2-tert-butylimino-2-diethylamino-1,3-dimethylperhydro-1,3,2-diazaphosphorine (0.506 g, 0.533 mL, 1.79 mmol) and the reaction mixture was stirred at room temperature overnight. The reaction mixture was diluted with water and acidifed with 2M HCl. The material was then extracted with ethyl acetate. The organic phase was concentrated *in vacuo.* The material was purified by flash chromatography (0 to 70% EtOAc in cyclohexane) to give a white solid. This material was crystallised from hot methanol to give 2-chloro-3-methylsulfanyl-N-(1-methyltetrazol-5-yl)-4-(2,2,2-trifluoroethoxy)benzamide (0.236 g, 0.544 mmol, 67%) as a white crystals. 1H NMR (Acetonitrile): 7.65 (d,1H), 7.11 (d,1H), 4.71 (q,2H), 4.01 (s,3H), 2.46 (s,3H).

### Example 2. Preparation of Compound 1.005.

### Step 1

To a 3 necked flask was added THF (22 mL) and the reaction mixture was purged and filled with N₂. Diisopropylamine (1.19 g, 1.65 mL, 11.6 mmol) was added. The reaction was stirred at -78 °C for 30 min. N-butyllithium in hexane (2.8 g, 2.5 mol/L, 4.1 mL, 10.2 mmol) was added dropwise. This was stirred for 1 h, then the mixture was allowed to warm to -40 °C, then cooled to -78 °C again. A solution of 1-bromo-2-chloro-4-(trifluoromethoxy)benzene (2 g, 7.26 mmol) in 5 mL of THF was added and the reaction mixture was stirred at -78 °C for 1.5 h. Dimethyl disulfide (1.38 g, 1.32 mL, 14.5 mmol) was added dropwise and the mixture was stirred at -78 °C for 1 h. The reaction mixture was quenched by adding it cold into a stirred solution of water. 2M HCl was added until the mixture was acidic and the mixture was stirred for 15 min. The material was extracted with diethyl ether and the organic phase was concentrated *in vacuo* to give 1-bromo-2-chloro-3-methylsulfanyl-4-(trifluoromethoxy)benzene (2.09 g, 6.50 mmol, 90%) as a colourless oil. 1H NMR (Chloroform): 7.62 (d,1H), 7.11 (d,1H), 2.46 (s,3H)

### Step 2

To a vessel containing NMP (47 mL) was added palladium(II) acetate (0.082 g, 0.364 mmol), XantPhos (0.434 g, 0.728 mmol), N-formylsaccharin (1.73 g, 8.19 mmol) and 1-bromo-2-chloro-3-methylsulfanyl-4-(trifluoromethoxy)benzene (1.17 g, 3.64 mmol). To a second vessel was added triethylamine (1.66 g, 2.28 mL, 16.4 mmol), NMP (47 mL) and water (2.34 mL). The reaction was carried out in a Uniqsis FlowSyn. The two solutions were pumped through a T-piece and then round a 20 mL stainless steel coil heated to 170 °C. The flow rate was set so that the total residence time was 20 mins. The reaction mixture was cooled to room temperature and was diluted with ethyl acetate. The organic phase was washed with 2M HCl, then with water. The organic phase was concentrated *in vacuo.* The material was purified by flash chromatography (0 to 100% EtOAc in cyclohexane) to give 2-chloro-3-methylsulfanyl-4-(trifluoromethoxy)benzoic acid (0.691 g, 2.41 mmol, 66%) as a yellow solid. 1H NMR (Methanol): 7.82 (d,1H), 7.44 (d,1H), 2.46 (s,3H)

### Step 3

To a flask containing 2-chloro-3-methylsulfanyl-4-(trifluoromethoxy)benzoic acid (0.125 g, 0.436 mmol) was added 2,3,4,5,6-pentafluorophenol (88.2 mg, 0.480 mmol) and DCM (2.5 mL). 1-(3-Dimethylaminopropyl)-3-ethylcarbodiimide hydrochloride (0.101 g, 0.501 mmol) was added and the reaction was stirred at room temperature for 1 h. The reaction mixture was diluted with DCM and washed with saturated aqueous NaHCO3. The organic phase was concentrated *in vacuo* to give (2,3,4,5,6-pentafluorophenyl) 2-chloro-3-methylsulfanyl-4-(trifluoromethoxy)benzoate (0.197 g, 0.436 mmol, 100%) as a pale yellow oil, which was used crude without further purification.

### Step 4

To a flask containing the (2,3,4,5,6-pentafluorophenyl) 3-amino-2-chloro-4-(trifluoromethyl)benzoate (0.197 g, 0.436 mmol) was added DMF (2 mL). 1-Methyltetrazol-5-amine (47.5 mg, 0.480 mmol) was added, followed by 2-tert-butylimino-2-diethylamino-1,3-dimethylperhydro-1,3,2-diazaphosphorine (0.271 g, 0.286 mL, 0.959 mmol) and the reaction mixture was stirred at room temperature overnight. The reaction mixture was diluted with water, and acidifed with 2M HCl. The material was then extracted with ethyl acetate. The organic phase was concentrated *in vacuo.* The material was purified by flash chromatography (0 to 100% EtOAc in cyclohexane) to give 2-chloro-3-methylsulfanyl-N-(1-methyltetrazol-5-yl)-4-(trifluoromethoxy)benzamide (76.6 mg, 0.208 mmol, 48%) as an off-white solid. 1H NMR (Methanol): 7.73 (d,1H), 7.52 (d,1H), 4.07 (s,3H), 2.48 (s,3H)

### Example 3. Preparation of Compound 2.001.

The synthesis of the starting material phenol is described in the procedure above for Compound 1.001.

### Step 1

To a flask containing 4-bromo-3-chloro-2-methylsulfanyl-phenol (2 g, 7.89 mmol) was added DMF (20 mL). K₂CO₃ (1.38 g, 9.47 mmol) was added, followed by sodium 2-chloro-2,2-difluoro-acetic acid (1.45 g, 9.47 mmol). The reaction mixture was stirred at 100 °C for 45 min, behind a blast shield. The mixture was cooled to room temperature. The reaction mixture was diluted with water and extracted with ethyl acetate. The organic phase was washed with water then concentrated *in vacuo.* The material was purified by flash chromatography (0 to 15% EtOAc in cyclohexane) to give a 1-bromo-2-chloro-4-(difluoromethoxy)-3-methylsulfanyl-benzene (1.46 g, 4.81 mmol, 61%) as a colourless oil. 1H NMR (Chloroform): 7.59 (d,1H), 7.02 (d,1H), 6.56 (t,1H), 2.46 (s,3H)

### Step 2

To a vessel containing NMP (55 mL) was added palladium(II) acetate (0.101 g, 0.451 mmol), XantPhos (0.538 g, 0.903 mmol), N-formylsaccharine (2.15 g, 10.1 mmol) and 1-bromo-2-chloro-4-(difluoromethoxy)-3-methylsulfanyl-benzene (1.37 g, 4.51 mmol). To a second vessel was added triethylamine (2.06 g, 2.83 mL, 20.3 mmol), NMP (55 mL) and water (2.74 mL). The reaction was carried out in a Uniqsis FlowSyn. The two solutions were pumped through a T-piece and then round a 20 mL stainless steel coil heated to 170 °C. The flow rate was set so that the total residence time was 20 min. The reaction mixture was cooled to room temperature and was diluted with ethyl acetate. The organic phase was washed with 2M HCl, then with water. The organic phase was concentrated *in vacuo.* The material was purified by flash chromatography (0 to 100% EtOAc in cyclohexane) to give 2-chloro-4-(difluoromethoxy)-3-methylsulfanyl-benzoic acid (0.953 g, 3.19 mmol, 70%) as a yellow solid. 1H NMR(Chloroform): 7.91 (d,1H), 7.18 (d,1H), 6.65 (t,1H), 2.47 (s,3H)

### Step 3

To a flask containing 2-chloro-4-(difluoromethoxy)-3-methylsulfanyl-benzoic acid (0.65 g, 2.42 mmol) was added 2,3,4,5,6-pentafluorophenol (0.668 g, 3.63 mmol) and DCM (13 mL). 1-(3-Dimethylaminopropyl)-3-ethylcarbodiimide hydrochloride (0.537 g, 2.66 mmol) was added and the reaction mixture was stirred at room temperature for 2 h. The reaction mixture was diluted with DCM and washed with saturated aqueous NaHCO3. The organic phase was concentrated *in vacuo* to give (2,3,4,5,6-pentafluorophenyl) 2-chloro-4-(difluoromethoxy)-3-methylsulfanyl-benzoate (1.3 g, 3.00 mmol, 124%) as a green oil, which was used crude without further purification.

### Step 4

To a flask containing (2,3,4,5,6-pentafluorophenyl) 3-[dicyclopropylmethylcarbamoyl(methoxy)amino]-2-methyl-4-methylsulfonyl-benzoate (0.526 g, 1.21 mmol) was added acetonitrile (10 mL). 5-Methyl-1,3,4-oxadiazol-2-amine (0.132 g, 1.33 mmol) was added, followed by 2-tert-butylimino-2-diethylamino-1,3-dimethylperhydro-1,3,2-diazaphosphorine (0.821 g, 0.866 mL, 2.90 mmol) and the reaction mixture was stirred at room temperature overnight. The reaction mixture was diluted with water and acidifed with 2M HCl. The material was then extracted with ethyl acetate. The organic phase was concentrated *in vacuo.*

The material was purified by flash chromatography (0 to 75% EtOAc in cyclohexane) to give 2-chloro-4-(difluoromethoxy)-N-(5-methyl-1,3,4-oxadiazol-2-yl)-3-methylsulfanyl-benzamide (0.150 g, 0.399 mmol, 33%) as an off-white solid. 1H NMR (Methanol): 7.67 (d,1H), 7.19 (d,1H), 6.64 (t,1H), 2.54 (s,3H), 2.46 (s,3H)

### Example 4. Preparation of compound 1.002

The starting material is the product of Step 3 from Example 3.

### Step 1

To a flask containing (2,3,4,5,6-pentafluorophenyl) 2-chloro-4-(difluoromethoxy)-3-methylsulfanyl-benzoate (0.23 g, 0.53 mmol) was added acetonitrile (4.6 mL), 1-methyltetrazol-5-amine (0.115 g, 1.16 mmol) and 2-tert-butylimino-2-diethylamino-1,3-dimethylperhydro-1,3,2-diazaphosphorine (0.35 mL, 1.2 mmol). The reaction mixture was stirred at RT for 30 min, then was concentrated in vacuo (room temp bath). The residue was diluted with water and washed with ethyl acetate. The aqueous phase was then acidified with 2M HCl and extracted with ethyl acetate x 2. The combined organic phases were dried (MgSO₄) and concentrated under reduced pressure. Flash chromatography (0 to 40 % ethyl acetate in cyclohexane) gave 2-chloro-4-(difluoromethoxy)-3-methylsulfanyl-N-(1-methyltetrazol-5-yl)benzamide (0.115 g, 0.329 mmol, 62%) as a white solid.

### Example 5. Preparation of compound 1.003

The starting material is compound 1.002, prepared in Example 4.

To a flask containing 2-chloro-N-(5-methyl-1,3,4-oxadiazol-2-yl)-3-methylsulfanyl-4-(trifluoromethoxy)benzamide (0.15 g, 0.4289 mmol) was added DCM (6 mL) and 3-chloroperoxybenzoic acid (0.24 g, 1.1 mmol). The reaction was stirred at RT for 16 h. A further aliquot of 3-chloroperoxybenzoic acid (0.10 g, 0.44 mmol) was added. After stirring for a further 2.5 h, the reaction mixture was quenched with addition of saturated aqueous sodium metabisulfite and the phases were separated. The aqueous phase was extracted with DCM and the combined organic layers were dried (MgSO₄) and concentrated under reduced pressure. Flash chromatography (0 to 80 % ethyl acetate in cyclohexane) gave 2-chloro-4-(difluoromethoxy)-3-methylsulfonyl-N-(1-methyltetrazol-5-yl)benzamide (0.100 g, 0.263 mmol, 61%) as a white solid.

### Example 6. Preparation of compound 1.009

The starting material was prepared in Step 3 from Example 1.

### Steps 1 and 2

To a solution of 4-bromo-3-chloro-2-methylsulfanyl-phenol (5.10 g, 20.1 mmol) in DMSO (50 mL) was added 1,2-dibromo-1,1,2,2-tetrafluoro-ethane (7.84 g, 30.2 mmol) and KOH (1.46 g, 26.1 mmol). The mixture was stirred at 70 C for 16 h. The reaction mixture was cooled to room temperature, then concentrated in vacuo. The crude material was diluted with water and extracted with ethyl acetate and the organic phase was concentrated in vacuo. The material was purified by flash chromatography (PE) to afford a mixture of 1-bromo-4-(2-bromo-1,1,2,2-tetrafluoro-ethoxy)-2-chloro-3-methylsulfanyl-benzene and 1-bromo-2-chloro-3-methylsulfanyl-4-(1,1,2,2-tetrafluoroethoxy)benzene (total 6.3 g) as a colorless oil.

This mixture was used crude in the following step:
To a mixture of 1-bromo-4-(2-bromo-1,1,2,2-tetrafluoro-ethoxy)-2-chloro-3-methylsulfanyl-benzene and 1-bromo-2-chloro-3-methylsulfanyl-4-(1,1,2,2-tetrafluoroethoxy)benzene (total 6.3 g) in AcOH (15 mL) was added Zn (3.81 g, 58.3 mmol). The mixture was stirred at 70 °C for 3 h. After cooling to room temperature, the crude material was diluted with water (80 ml) and extracted with ethyl acetate (50 ml) and the organic phase was washed with sodium bicarbonate solution (20ml x 3) and concentrated in vacuo to afford 1-bromo-2-chloro-3-methylsulfanyl-4-(1,1,2,2-tetrafluoroethoxy)benzene (3.90 g, 11.0 mmol, 2 steps yield: 55%) as a colorless oil.

### Step 3

To a solution of 1-bromo-2-chloro-3-methylsulfanyl-4-(1,1,2,2-tetrafluoroethoxy)benzene (10.2 g, 28.8 mmol) in ethanol (60 mL) was added Pd(OAc)₂ (0.130 g, 0.577 mmol) and 1,1'-bis(diphenylphosphino)ferrocene (0.800 g, 1.44 mmol) . The mixture was charged with CO (2.0 MPa) and stirred at 120 °C for 6 h. After cooling to room temperature, the crude material was concentrated in vacuo and purified by flash chromatography (petroleum ether : ethyl acetate 40:1 to 20:1) to afford ethyl 2-chloro-3-methylsulfanyl-4-(1,1,2,2-tetrafluoroethoxy)benzoate (8.00 g, 23.1 mmol, 80 %) as a yellow liquid.

### Step 4

To a solution of ethyl 2-chloro-3-methylsulfanyl-4-(1,1,2,2-tetrafluoroethoxy)benzoate (15.0 g, 43.3 mmol) in THF (30 mL) and water (30 ml) was added LiOH·H₂O (5.45 g, 130 mmol). The mixture was stirred at room temperature and stirred for 16 h. Dilute hydrochloric acid was added to adjust pH to 2. The mixture was extracted with ethyl acetate (50 ml) and the organic phase was concentrated in vacuo and purified by flash chromatography (pet. Ether : ethyl acetate 2:1 to 1:1) to afford 2-chloro-3-methylsulfanyl-4-(1,1,2,2-tetrafluoroethoxy)benzoic acid (11.5 g, 36.1 mmol, 83%) as a white solid.

### Step 5

To a stirred suspension of 2-chloro-3-methylsulfanyl-4-(1,1,2,2-tetrafluoroethoxy)benzoic acid (2.00 g, 6.28 mmol) and 2,3,4,5,6-pentafluorophenol (1.27 g, 6.90 mmol) in dichloromethane (30 mL) at room temperature was added 3-(ethyliminomethyleneamino)-N,N-dimethyl-propan-1-amine hydrochloride (1.44 g, 7.51 mmol). The mixture was stirred at room temperature. After 5 minutes of adding EDC, the mixture was a homogeneous solution.

The reaction mixture was stirred overnight at room temperature. The reaction was quenched by addition of sat. aq. NaHCO3 (100 mL). The mixture was stirred at room temperature for a further 5 minutes. The mixture filtered through a phase separation cartridge and the organics are collected. The filtrate was adsorbed onto silica and the crude product was purified by flash column chromatography (0-10% gradient of EtOAc in cyclohexane) to afford (2,3,4,5,6-pentafluorophenyl) 2-chloro-3-methylsulfanyl-4-(1,1,2,2-tetrafluoroethoxy)benzoate (3.42 g, 7.06 mmol) as a pale yellow oil, which crystallised on standing.

### Step 6

To a stirred solution of (2,3,4,5,6-pentafluorophenyl) 2-chloro-3-methylsulfanyl-4-(1,1,2,2-tetrafluoroethoxy)benzoate (0.500 g, 1.03 mmol) in acetonitrile (10 mL) at room temperature was added 1-methyltetrazol-5-amine (0.225 g, 2.270 mmol) followed by 2-tert-butylimino-N,N-diethyl-1,3-dimethyl-1,3,2-diazaphosphinan-2-amine (0.64 g, 0.68 mL, 2.3 mmol). The mixture was stirred at room temperature overnight. The reaction was quenched by addition of 2 M aq. HCl (100 mL). The mixture was stirred at room temperature for a further 5 minutes. The mixture was diluted with EtOAc (100 mL). The phases were separated. The aqueous phase was extracted with EtOAc (100 mL). The combined organic phases were washed with brine (100 mL), dried (MgSO4) and purified by reverse phase chromatography to give 2-chloro-3-methylsulfanyl-N-(1-methyltetrazol-5-yl)-4-(1,1,2,2-tetrafluoroethoxy)benzamide (295 mg, 0.701 mmol, 68%). 1H NMR (400 MHz, d4-methanol): 2.45 (s, 3 H) 4.07 (s, 3 H) 6.30 - 6.63 (m, 1 H) 7.49 - 7.55 (m, 1 H) 7.66 - 7.75 (m, 1 H).

### Example 7: Preparation of Compound 1.010

The starting material is the same as produced in Step 4 of Example 6.

### Step 1

To a flask containing 2-chloro-3-methylsulfanyl-4-(1,1,2,2-tetrafluoroethoxy)benzoic acid (3.00 g, 9.41 mmol) was added dichloromethane (90 mL) and 3-chloroperoxybenzoic acid (6.32 g, 28.2 mmol). The reaction was stirred for 16 h at RT. The reaction mixture was quenched with saturated aqueous sodium metabisulfite and the phases were separated. The aqueous layer was extracted with ethyl acetate. The combined organic phases were concentrated and purified by flash chromatography to give 2-chloro-3-methylsulfonyl-4-(1,1,2,2-tetrafluoroethoxy)benzoic acid (2.46 g, 75%) as a white solid.

### Step 2

To a stirred suspension of 2-chloro-3-methylsulfonyl-4-(1,1,2,2-tetrafluoroethoxy)benzoic acid (2.5 g, 7.1 mmol) and 2,3,4,5,6-pentafluorophenol (1.4 g, 7.6 mmol) in dichloromethane (30 mL) at room temperature was added 3-(ethyliminomethyleneamino)-N,N-dimethyl-propan-1-amine hydrochloride (1.6 g, 8.3 mmol). Initially heterogeneous, however, within 5 minutes of adding EDC, the mixture was a homogeneous solution. The mixture was stirred at room temperature for 3 hours. The reaction was quenched by addition of sat. aq. NaHCO3 (100 mL). The mixture was stirred at room temperature for a further 5 minutes. The filtrate was adsorbed onto silica and the crude product was purified by flash column chromatography (0-10% gradient of EtOAc in cyclohexane) to give (2,3,4,5,6-pentafluorophenyl) 2-chloro-3-methylsulfonyl-4-(1,1,2,2-tetrafluoroethoxy) benzoate (3.42 g, 6.62 mmol, 93%) as a colourless oil.

### Step 3

To a stirred solution of (2,3,4,5,6-pentafluorophenyl) 2-chloro-3-methylsulfonyl-4-(1,1,2,2-tetrafluoroethoxy)benzoate (A, 300 mg, 0.5806 mmol, 100 mass%) in acetonitrile (8 mL) at room temperature was added 1-methyltetrazol-5-amine (0.127 g, 1.28 mmol) followed by 2-tert-butylimino-N,N-diethyl-1,3-dimethyl-1,3,2-diazaphosphinan-2-amine (0.36 g, 0.38 mL, 1.3 mmol). The mixture was stirred at room temperature for 2 h. The reaction was quenched by addition of 2 M aq. HCl (10 mL). The mixture was stirred at room temperature for a further 5 minutes. The mixture was diluted with EtOAc (20 mL). The phases were separated. The aqueous phase was extracted with EtOAc (10 mL). The combined organic phases were adsorbed onto C18-silica and the crude product was purified by reverse phase chromatography. To give 2-chloro-3-methylsulfonyl-N-(1-methyltetrazol-5-yl)-4-(1,1,2,2-tetrafluoroethoxy)benzamide (170 mg, 0.374 mmol, 64%) as a white solid.

### Example 8: Preparation of Compound 1.007

The starting material is the product of Step 3 in Example 1.

### Step 1

A solution of 4-bromo-3-chloro-2-methylsulfanyl-phenol (2.50 g, 9.86 mmol) in sodium hydroxide (5% solution in water) (8.87 mL) was added to a cooled (ice bath) solution of thiocarbonyl dichloride (9.86 mmol, 0.752 mL, 1.13 g) in chloroform (6 mL). The reaction mixture was scrubbed through bleach and stirred at 0 °C for 2.5 h. The phases were separated. The organic layer was washed (aq. 2M HCl then water), dried (MgSO4) and concentrated under vacuum to give O-(4-bromo-3-chloro-2-methylsulfanyl-phenyl) chloromethanethioate (3.07 g, 9.24 mmol, 94%) as a yellow liquid. 1H NMR (400 MHz, CDCl₃) δ = 7.69 (d, J = 8.7 Hz, 1H), 6.98 (d, J = 8.8 Hz, 1H), 2.44 (s, 3H).

### Step 2

An oven dried flask was evacuated and purged with nitrogen (x3). A solution of O-(4-bromo-3-chloro-2-methylsulfanyl-phenyl) chloromethanethioate (3.00 g, 9.03 mmol) in THF (90 mL) was added followed by Copper(I) cyanide di(lithium chloride) complex solution (1M in THF, 9.94 mL, 9.94 mmol). It was cooled to -78 °C. The methyl magnesium bromide (3M solution in THF) (9.94 mmol, 3.31 mL) was added slowly (the temperature was maintained below - 70 °C during the addition). After the addition was complete it was stirred at -78 °C for 1 h. The reaction mixture was warmed to 0 °C and it was stirred at this temperature for 1 h. The reaction was quenched by the addition of sat. aq. NH₄Cl. It was extracted with EtOAc (x3). The combined EtOAc extracts were dried (MgSO₄) and concentrated under vacuum. The residue was purified by chromatography (0 to 10% EtOAc in cyclohexane) to give O-(4-bromo-3-chloro-2-methylsulfanyl-phenyl) ethanethioate (1.5 g, 4.8 mmol, 53%) as a yellow oil. 1H NMR (400 MHz, CDCl₃) δ = 7.65 (d, J = 8.7 Hz, 1H), 6.86 (d, J = 8.7 Hz, 1H), 2.38 (s, 3H)

### Step 3

A solution of O-(4-bromo-3-chloro-2-methylsulfanyl-phenyl) ethanethioate (1.5 g, 4.8 mmol) in dichloromethane (19 mL) was stirred under nitrogen. This solution was treated with antimony(III) chloride (0.24 mmol, 0.055 g) then Deoxo-Fluor 50% solution in toluene (6.7 mmol, 3.4 mL). The reaction mixture was stirred at RT for 24 h under a blanket of nitrogen. The reaction mixture was quenched by the addition of aq. sat. NaHCO₃. This was extracted with EtOAc (x3). The combined EtOAc extracts were dried (MgSO₄) and concentrated under vacuum. The residue was purified by chromatography (0 to 10% EtOAc in cyclohexane) to give 1-bromo-2-chloro-4-(1,1-difluoroethoxy)-3-methylsulfanyl-benzene (0.867 g, 2.73 mmol, 57% yield). 1HNMR (400 MHz, CDCl3) δ = 7.56 (d, J = 8.9 Hz, 1H), 7.15 (td, J = 1.3, 8.9 Hz, 1H), 2.42 (s, 3H), 2.00 (t, J = 13.4 Hz, 3H).

### Step 4

To a vessel containing NMP (20 mL) was added palladium(II) acetate (74 mg, 0.33 mmol), XantPhos (39 mg, 0.66 mmol), N-formylsaccharine (1.57 g, 7.44 mmol) and 1-bromo-2-chloro-4-(1,1-difluoroethoxy)-3-methylsulfanyl-benzene (1.05 g, 3.31 mmol). To a second vessel was added triethylamine (2.07 mL, 14.9 mmol), NMP (20 mL) and water (2.1 mL). The reaction was carried out in a Uniqsis FlowSyn. The two solutions were pumped through a T-piece and then round a 20 mL stainless steel coil heated to 170 °C. The flow rate was set so that the total residence time was 15 min. The reaction mixture was cooled to room temperature and was diluted with ethyl acetate. The organic phase was washed with 2M HCl, then with water. The organic phase was concentrated in vacuo. The material was purified by reversed phase flash chromatography to give 2-chloro-4-(1,1-difluoroethoxy)-3-methylsulfanyl-benzoic acid (0.565 g, 2.00 mmol, 60%) as a yellow solid. 1H NMR (d4-methanol): 7.74 (d, 1H), 7.39 (m, 1H), 2.43 (s, 3H), 2.03 (m, 3H).

### Step 5

To a flask containing 2-chloro-4-(1,1-difluoroethoxy)-3-methylsulfanyl-benzoic acid (0.565 g, 2.00 mmol) was added: dichloromethane (11 mL) and 2,3,4,5,6-pentafluorophenol (0.405 g, 2.20 mmol). 3-(ethyliminomethyleneamino)-N,N-dimethyl-propan-1-amine hydrochloride (0.464 g, 2.30 mmol) was added and the reaction was stirred for 1 h. The reaction mixture was quenched by addition of saturated aqueous saturated aqueous sodium bicarbonate and the phases were separated and extracted with ethyl acetate x 2. The organic phase were combined dried (MgSO₄) and concentrated in vacuo. The crude material was purified by flash chromatography (0 to 10 % ethyl acetate in cyclohexane) to give (2,3,4,5,6-pentafluorophenyl) 2-chloro-4-(1,1-difluoroethoxy)-3-methylsulfanyl-benzoate (604 mg, 1.23 mmol, 63%) as a yellow solid. 1H NMR (CDCl₃): 8.01 (d, 1H), 7.45 (m, 1H), 2.45 (s, 3H), 2.06 (m, 3H).

### Step 6

To a flask containing (2,3,4,5,6-pentafluorophenyl) 2-chloro-4-(1,1-difluoroethoxy)-3-methylsulfanyl-benzoate (0.400 g, 0.892 mmol) was added DCM (4 mL) and 3-chlorobenzenecarboperoxoic acid (0.528 g, 2.14 mmol). After stirring for 5 h, a further portion of 3-chlorobenzenecarboperoxoic acid (0.220 g, 0.892 mmol) was added. After stirring for 24 h, the reaction mixture was quenched by the addition of saturated aqueous sodium metabisulfite. The phases were separated, and the aqueous layer was extracted with DCM. The combined organic layers were washed with saturated aqueous sodium carbonate x 2, then dried (MgSO₄) and concentrated in vacuo to give (2,3,4,5,6-pentafluorophenyl) 2-chloro-4-(1,1-difluoroethoxy)-3-methylsulfonyl-benzoate (0.411 g, 0.8550 mmol, 96%) as pale yellow crystals. ¹H NMR (CDCl₃): 8.14 (d, 1H), 7.58 (m, 1H), 3.36 (s, 3H), 2.08 (m, 3H).

### Step 7

To a flask containing (2,3,4,5,6-pentafluorophenyl) 2-chloro-4-(1,1-difluoroethoxy)-3-methylsulfanyl-benzoate (0.21 g, 0.4369 mmol) was added acetonitrile (4.2 mL), 1-methyltetrazol-5-amine (0.09524 g, 0.9611 mmol) and 2-tert-butylimino-N,N-diethyl-1,3-dimethyl-1,3,2-diazaphosphinan-2-amine (0.272 g, 0.287 mL, 0.961 mmol) were then added and stirred at RT for 1 h. The reaction mixture was concentrated in vacuo, then diluted with 2M aq HCl and extracted with ethyl acetate. The organic phase was dried (MgSO₄)₄, concentrated and purified by reversed phase chromatography to give 2-chloro-3-methylsulfonyl-N-(1-methyltetrazol-5-yl)-4-(1,1,2,2-tetrafluoroethoxy)benzamide (170 mg, 0.374 mmol, 64%) as a white solid.

**TABLE 1 - Examples of herbicidal compounds of the present invention.**

| **COMPOUND** | **STRUCTURE** | **NMR** |
|---|---|---|
| 1.001 | 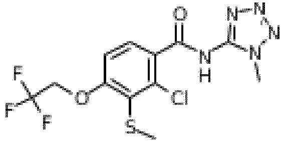 | ¹H NMR(d-MeCN): 7.65(d, 1H), 7.11(d, 1H), 4.71 (q, 2H), 4.01 (s, 3H), 2.46 (s, 3H) |
| 1.002 | 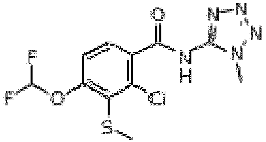 | ¹H NMR(CDCl₃): 10.19-11.00 (m,1H), 7.69 (d,1H), 7.25 (d,1H), 6.67 (t,1H), 4.12 (s,3H), 2.49 (s,3H) |
| 1.003 | 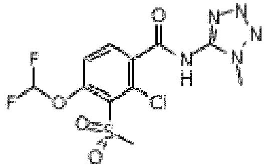 | ¹H NMR(d4-MeOD): 7.95(d,1H), 7.53 (d,1H), 7.01 (t,1H), 4.07(s,3H), 3.41(s,3H) |
| 1.004 | 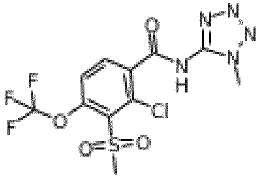 | ¹H NMR(CDCl₃): 7.87(d,1H), 7.53 (m,1H), 4.15(s,3H), 3.40(s,3H) |
| 1.005 | 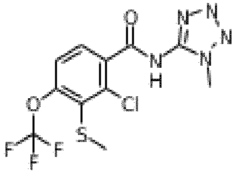 | ¹H NMR(d4-MeOD): 7.73 (d,1H), 7.52 (m,1H), 4.07(s,3H), 2.48(s,3H) |
| 1.006 | 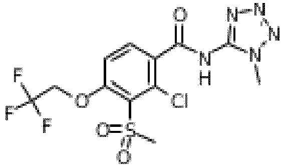 | ¹H NMR (Acetonitrile): 7.86 (d, 1H), 7.30 (d, 1H), 4.78 (q, 2H), 4.02 (s, 3H), 3.36 (s, 3H) |
| 1.007 | 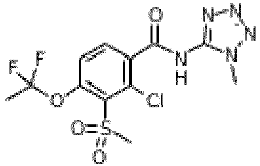 | ¹H NMR(d4 - MeOD): 7.97(d,1H), 7.67(d,1H), 4.10(s,3H), 3.40(s,3H), 2.07(m,3H) |
| 1.008 | 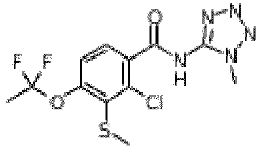 | ¹H NMR(d4-MeOD) 7.67(d,1H),7.49(m,1H), 4.09(s,3H),2.46(s,3H), 2.06(m,3H) |
| 1.009 | 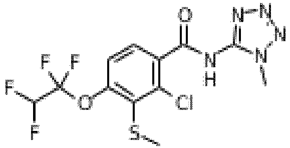 | ¹H NMR (d4-MeOD) δ ppm 2.45 (s, 3 H) 4.07 (s, 3 H) 6.30 - 6.63 (m, 1 H) 7.49 - 7.55 (m, 1 H) 7.66 -7.75 (m, 1 H) |
| 1.010 | 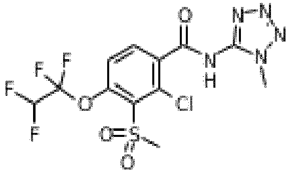 | ¹H NMR (400 MHz, methanol) δ ppm 3.40 (s, 3 H) 4.08 (s, 3 H) 6.23 - 6.56 (m, 1 H) 7.65 - 7.74 (m, 1 H) 7.98 - 8.07 (m, 1 H) |
| 1.011 | 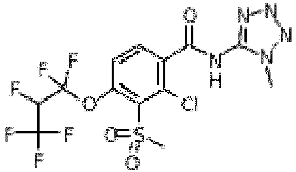 | |
| 1.012 | 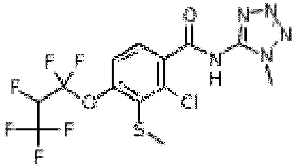 | |
| 1.013 | 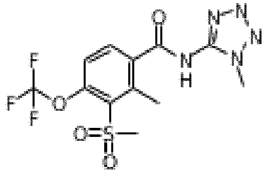 | ¹H NMR (d-MeCN): 7.90 (d,1H), 7.55 (d,1H), 4.01 (s,3H), 3.30 (s,3H), 2.81 (s,3H) |
| 1.014 | 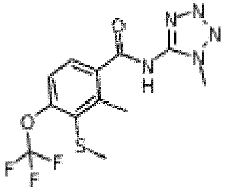 | ¹H NMR (d4-MeOD): 7.68 (d,1H), 7.37 (d,1H), 4.05 (s,3H), 2.72 (s,3H), 2.36 (s,3H) |
| 1.015 | 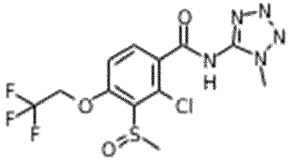 | ¹HNMR(Acetonitrile): 7.81 (d,1H), 7.22 (d,1H), 4.69-4.82 (m,2H), 4.00 (s,3H), 3.08 (s,3H) |
| 1.016 | 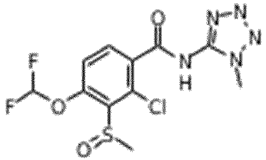 | ¹H NMR (Methanol): 7.90 (d, 1H), 7.49 (d, 1H), 7.23 - 6.87 (m, 1H), 4.05 (s, 3H), 3.18 (s, 3H) |
| 1.017 | 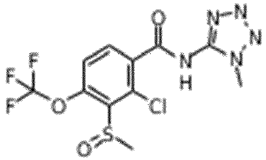 | ¹HNMR(d4-MeOD): 7.96 (d,1H), 7.59-7.70 (m,1H), 4.07 (s,3H), 3.17 (s,3H) |
| 1.018 | 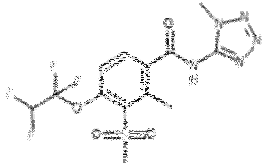 | ¹H NMR (400 MHz, methanol) δ ppm 2.81 (s, 3 H) 4.04 - 4.08 (m, 3 H) 6.24 - 6.58 (m, 1 H) 7.57 - 7.62 (m, 1 H) 7.89 - 7.98 (m, 1 H) |
| 1.019 | 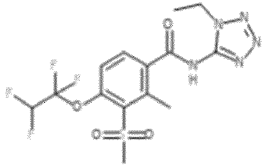 | ¹H NMR (400 MHz, methanol) δ ppm 1.58 (t, 3 H) 2.77 - 2.86 (m, 3 H) 4.40 (q, J=7.30 Hz, 2 H) 6.25 - 6.58 (m, 1 H) 7.54 - 7.62 (m, 1 H) 7.88 - 7.95 (m, 1 H) |
| 1.020 | 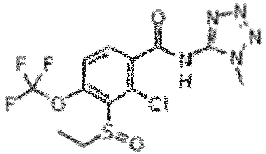 | ¹H NMR (DMSO-d6)11.95(brs,1H)8.07 (brd,1H) 7.73(brd,1H) 4.01(s,3H) 3.37-3.45(m,1H) 3.28-3.31(m,1H)1.14-1.30(m,3H) |
| 1.021 | 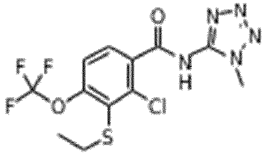 | ¹H NMR (DMSO-d6) δ ppm 1.11 - 1.18 (m, 3 H), 2.97 (q, 2 H) 4.01 (s, 3 H) 7.66 (br d, J=7.53Hz, 1 H) 7.89 (br d, J=8.78 Hz, 1 H) 11.88 (br s, 1 H). |
| 1.022 | 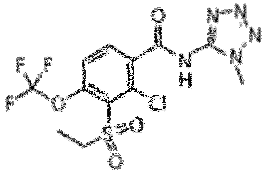 | ¹H NMR (DMSO-d6): 12.00 (br s, 1 H), 8.21 (d, 1 H), 7.82 (d, 1 H), 4.02 (s, 3 H), 3.65 - 3.55 (m, 2 H), 1.23 (t,3 H) |
| 1.023 | 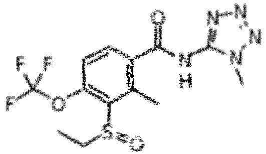 | ¹H NMR (DMSO-d6): 11.76 (s, 1 H), 7.92 (d, 1 H), 7.54 (d, 1 H), 4.01 (s, 3 H), 3.42 - 3.30 (m, 1 H), 3.23 - 3.10 (m, 1 H), 2.69 (s, 3 H), 1.21 (t, 3 H) |
| 1.024 | 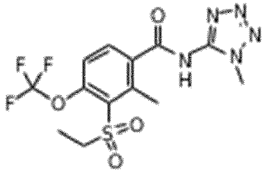 | ¹H NMR (DMSO-d6): 11.80 (br s, 1 H), 8.05 (d, 1 H), 7.66 (d, 1 H), 4.02 (s, 3 H), 3.51 - 3.42 (m, 2 H), 2.73 (s, 3 H), 2.21 (t, 3 H) |
| 1.025 | 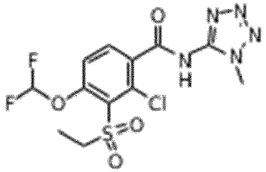 | ¹H NMR (DMSO-d6): 11.96 (br s, 1 H), 8.12 (d, 1 H), 7.60 - 7.22 (m, 2 H), 4.02 (s, 3 H), 3.59 - 3.49 (m, 2 H), 1.28 - 1.19 (m, 3 H) |
| 1.026 | 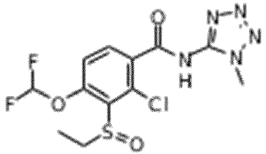 | ¹H NMR (DMSO-d6): 11.92 (br s, 1 H), 8.00 (d, 1 H), 7.60 - 7.15 (m, 2 H), 4.01 (s, 3 H), 3.50 - 3.25 (m, 2 H), 1.19 (t, 3 H) |
| 1.027 | 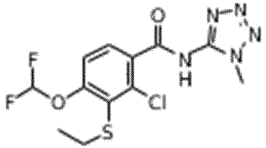 | ¹H NMR (DMSO-d6): 11.83 (br s, 1 H), 7.81 (d, 1 H), 7.62 - 7.26 (m, 2 H), 4.01 (s, 3 H), 3.00 - 2.90 (m, 2 H), 1.14 (t, 3 H) |
| 1.028 | 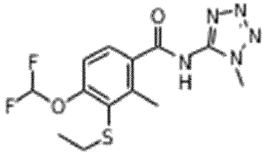 | ¹H NMR (DMSO-d6): 11.58 (br s, 1 H), 7.70 (d, 1 H), 7.57 - 7.20 (m, 2 H), 3.99 (s, 3 H), 2.88 - 2.80 (m, 2 H), 2.61 (s, 3 H), 1.12 (t, 3 H) |
| 1.029 | 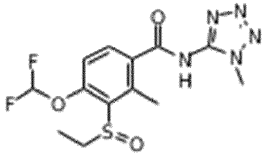 | ¹H NMR (DMSO-d6): 11.69(brs,1H) 7.85(brd,1H)7.54-7.17(m,2H)4.00(s,3H) 3.19(m,1H)3.17(m,1H)2.68(s,3H)1.20(brt,3H) |
| 1.030 | 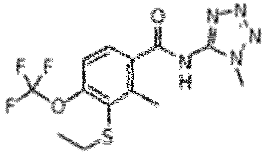 | ¹H NMR (DMSO-d6): 11.67 (s,1H), 7.77 (d,1H), 7.49 (brd, 1H), 4.01 (s,3H), 2.84 (q,2H), 2.64 (s,3H), 1.13 (t,3H) |
| 1.031 | 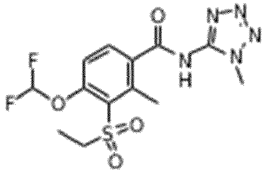 | ¹H NMR (d6-DMSO): 11.76 (1H, s), 7.98 (1H, d), 7.44 (1H, d), 7.37 (1H, t), 4.01 (3H, s), 3.47 (2H, q), 2.70 (3H, s), 1.21 (3H, t). |
| 1.032 | 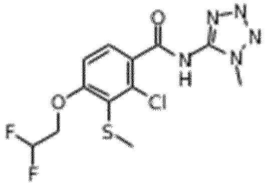 | ¹H NMR(Methanol): 7.61 (d,1H), 7.15 (d,1H), 6.14-6.46(m, 1H), 4.41 (td, 2H), 4.05 (s,3H), 2.43(s,3H) |
| 1.033 | 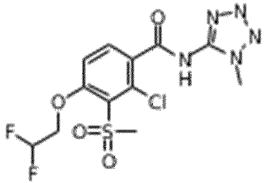 | ¹H NMR(Methanol): 7.86 (d,1H), 7.41 (d, 1H), 6.16-6.53 (m, 1H), 4.54 (td, 2H), 4.06 (s,3H), 3.39 (s,3H) |
| 1.034 | 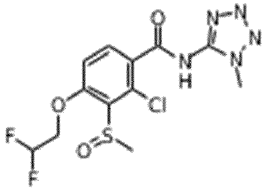 | ¹H NMR(Methanol): 7.85(d,1H), 7.36 (d, 1H), 6.18-6.52 (m, 1H), 4.46-4.57 (m, 3H), 4.04 (s, 3H), 3.18 (s, 3H) |
| 1.035 | 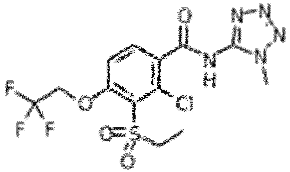 | ¹H NMR (400 MHz, DMSO-d6) δ ppm 1.19 (t, 3 H) 3.48 (q, 2 H) 4.00 (s, 3 H) 5.07 (q, 2 H) 7.50 (d, 1 H) 8.03 (d, 1 H) 11.85 (br s, 1 H) |
| 1.036 | 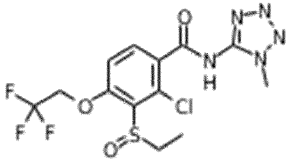 | ¹H NMR (400 MHz, DMSO-d6) δ ppm 1.14 (t, 3 H) 3.41 (q, 2H) 3.99 (s, 3 H) 5.07 (q, 2 H) 7.43 (d, 1 H) 7.93 (d, 1 H), 11.80(br s,1H) |
| 1.037 | 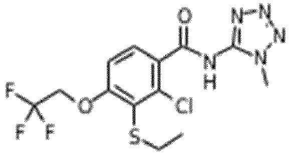 | ¹H NMR (400 MHz, DMSO-d6) δ ppm 1.10 (t, 3 H) 2.91 (q, 2 H) 3.99 (s, 3 H) 4.98 (q, 2 H) 7.30 (d, 1 H) 7.74 (d, 1 H) 11.70 (s, 1 H) |
| 1.038 | 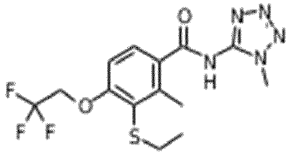 | ¹H NMR (400 MHz, DMSO-d6) δ ppm 1.07 (t, 3 H) 2.58 (s, 3 H) 2.82 (q, 2 H) 3.97 (s, 3 H) 4.92 (q, 2 H) 7.15 (d, 1 H) 7.67 (d, 1 H) 11.47 (s, 1 H) |
| 1.039 | 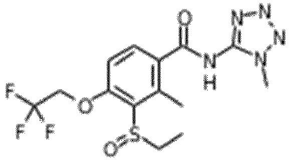 | ¹H NMR (400 MHz, DMSO-d6) δ ppm 1.16 (t, 3 H) 2.68 (s, 3 H) 3.10 - 3.30 (m, 2 H) 3.98 (s, 3 H) 4.96 (q, 2 H) 7.25 (d, 1 H) 7.80 (d, 1 H) 11.59 (br s, 1 H) |
| 1.040 | 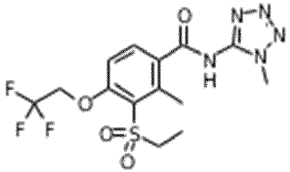 | ¹H NMR (400 MHz, DMSO-d6) δ ppm 1.17 (t, 3 H) 2.67 (s, 3 H) 3.41 (q, 2 H) 4.00 (s, 3 H) 5.03 (q,2 H) 7.37(d, 1 H) 7.91 (d, 1 H) 11.66 (br s, 1 H) |
| 1.041 | 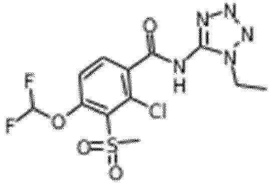 | ¹H NMR (Methanol) δ: 1.58 (t, 3H), 3.41 (s, 3H), 4.43 (q, 2H), 7.01 (t, 1H), 7.53 (d, 1H), 7.94 (d, 1H) |
| 1.042 | 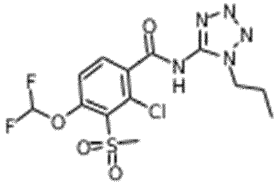 | ¹H NMR (Methanol): 7.94 (d, 1H), 7.54 (d,1H), 6.80-7.22 (m,1H), 4.37 (t,2H), 3.41(s,3H), 1.92-2.07(m,2H), 0.98(t,3H) |
| 1.043 | 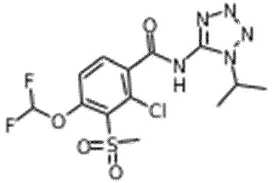 | ¹H NMR (Methanol): 7.96 (d, 1H), 7.54 (d, 1H), 6.77-7.28 (m, 1H), 4.81 (m, 1H), 3.41 (s, 3H), 1.62 (d, 6H) |
| 1.044 | 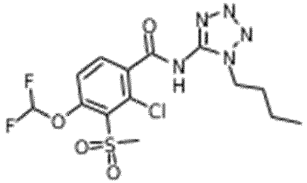 | ¹H NMR(Methanol): 7.93 (d, 1H), 7.54 (d, 1H), 6.80-7.24 (m,1H), 4.41 (t,2H), 3.41 (s,3H), 1.89-2.02 (m,2H), 1.28-1.50 (m,2H), 0.98 (t,3H) |
| 1.045 | 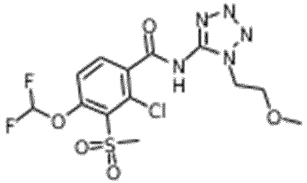 | ¹H NMR(Acetonitrile): 7.89(d,1H), 7.47(d, 1H), 6.61-7.24 (m,1H), 4.56 (t,2H), 3.81(t, 2H), 3.38 (s,3H), 3.31(s,3H) |
| 1.046 | 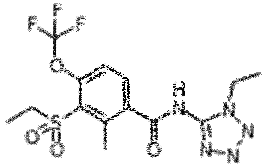 | ¹H NMR (400MHz, DMSO-d6): 11.73 (brs, 1H), 8.04 (d, 1H), 7.65 (d, 1H), 4.36 (q, 2H), 3.43-3.50 (m, 2H), 2.73 (s, 3H), 1.48 (t,3H), 1.21 (t, 3H) |
| 1.047 | 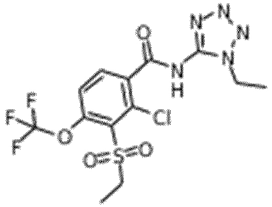 | ¹H NMR (DMSO-d6): 1.24 (t, 3 H), 1.48 (t, 3 H), 3.60 (q, 2 H), 4.38 (q, 2 H), 7.82 (d, 1 H), 8.21 (d, 1 H), 11.90 (brs, 1 H) |
| 1.048 | 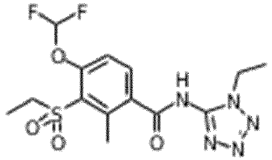 | ¹H NMR (400 MHz, DMSO-d6): 1.21 (t, 3 H), 1.48 (t, 3 H), 2.70 (s, 3 H), 3.38 - 3.51 (m, 2 H), 4.35 (q, 2 H), 7.36 (t, 1 H), 7.43 (d, 1 H), 7.96 (d, 1 H), 11.50 - 11.78 (br s, 1 H) |
| 1.049 | 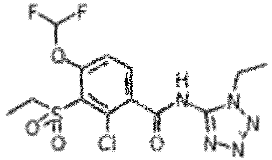 | ¹H NMR (400 MHz, DMSO-d6): 1.23 (t, 3 H), 1.48 (t, 3 H), 3.50 - 3.59 (m, 2 H), 4.38 (q, 2 H), 7.53 - 7.63 (m, 1 H), 7.88 - 7.94 (m, 1 H), 8.11 (br d, 1 H), 11.74 - 11.99 (br s, 1 H) |
| 1.050 | 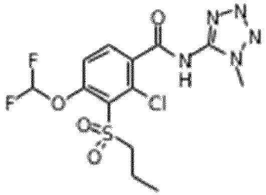 | ¹H NMR (400 MHz, DMSO-d6): 0.99 (t, 3 H), 1.70 (m, 2 H), 3.49 - 3.56 (m, 2 H), 4.02 (s, 3 H), 7.40 (s, 1 H), 7.53 -7.63 (m, 1 H), 8.11 (br d, 1H), 11.94 (br s, 1H) |
| 1.051 | 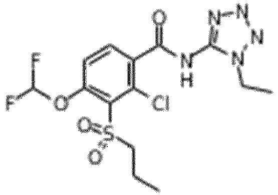 | ¹H NMR (400 MHz, DMSO-d6): 0.99 (t, 3 H), 1.48 (t, 3 H), 1.63 - 1.76 (m, 2 H), 3.49 - 3.56 (m, 2 H), 4.38 (q, 2 H),7.22 (s, 1 H), 7.53 - 7.63 (m, 1 H), 8.11 (br d, 1 H), 11.84 (br s, 1 H) |
| 1.052 | 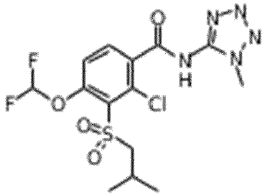 | ¹H NMR (400 MHz, DMSO-d6): 0.99 (t, 3 H), 1.48 (t, 3 H), 1.63 - 1.76 (m, 2 H), 3.49 - 3.56 (m, 2 H), 4.38 (q, 2 H),7.22 (s, 1 H), 7.53 - 7.63 (m, 1 H), 8.11 (br d, 1 H), 11.84 (br s, 1 H) |
| 1.053 | 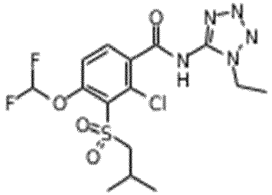 | ¹H NMR (d6-DMSO): 8.08 (1H, d), 7.60-7.19 (2H, m), 4.36 (2H, q), 3.47 (2H, d), 2.16 (1H, m), 1.47 (3H, t), 1.03 (6H, d) |
| 1.054 | 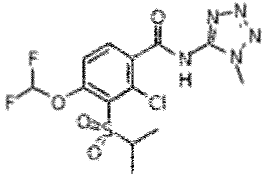 | ¹H NMR (400 MHz, CDCl3):1.39 - 1.44 (m, 6 H), 3.68 - 3.74 (m, 1 H), 4.14 (s, 3 H), 6.42 - 6.82 (m, 1 H), 7.42 - 7.50 (m, 1 H), 7.81 - 7.88 (m, 1 H) |
| 1.055 | 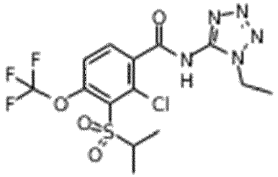 | ¹H NMR, (d4- methanol): 8.05 (br d, 1H), 7.71 (d, 1H), 4.44 (q, 2H), 3.78 (m, 1H), 1.59 (t, 3H), 1.36 (d, 6H) |
| 1.056 | 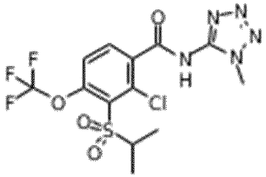 | ¹H NMR (d4-methanol): 8.07 (br d, 1H), 7.73 (br d, 1H), 4.10 (s, 3H), 3.80 (m, 1H), 1.38 (d, 6H) |
| 1.057 | 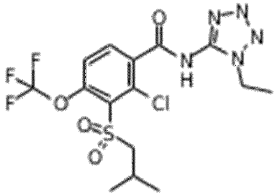 | ¹H NMR, (d4-methanol): 8.03 (d, 1H), 7.74 - 7.66 (m, 1H), 4.43 (q, 2H), 3.43 (d, 2H), 2.39 - 2.27 (m, 1H), 1.58 (t, 3H), 1.12 (d, 6H) |
| 1.058 | 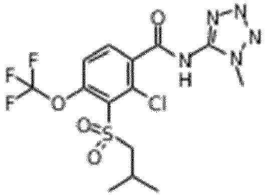 | ¹H NMR, (d4-methanol): 8.04 (d, 1H), 7.74 - 7.65 (m, 1H), 4.08 (s, 3H), 3.43 (d, 2H), 2.41 - 2.25 (m, 1H), 1.12 (d, 6H) |
| 1.059 | 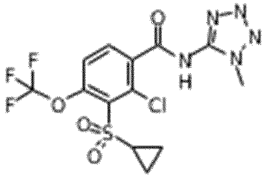 | ¹H NMR (d4-methanol): 8.04 (d, 1H), 7.72 (m, 1H), 4.10 (s, 3H), 3.24 (m, 1H), 1.42-1.35 (m, 2H), 1.23-1.15 (m, 2H) |
| 1.060 | 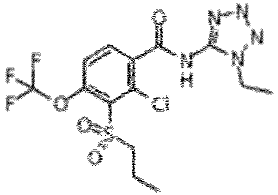 | ¹H NMR (400MHz, CDCl3): 7.86 (d, 1H), 7.49 (br d, 1H), 4.49 (q, 2H), 3.46-3.38 (m, 2H), 1.98-1.79 (m, 2H), 1.61 (t, 3H), 1.09 (t, 3H) |
| 1.061 | 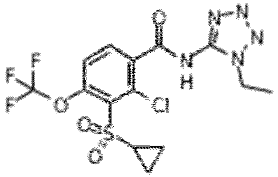 | ¹H NMR (d4-Methanol): 8.04 (d, 1H), 7.72 (m, 1H), 4.46 (m, 2H), 3.24 (m, 1H), 1.61 (m, 3H), 1.43-1.34 (m, 2H), 1.24-1.15 (m, 2H) |
| 1.062 | 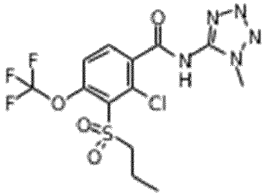 | ¹H NMR (400MHz, CDCl3): 7.90-7.86 (m, 1H), 7.54-7.49 (m, 1H), 4.13 (s, 3H), 3.44-3.38 (m, 2H), 1.95-1.85 (m, 2H), 1.10 (t, 3H) |
| 1.063 | 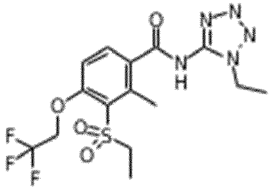 | ¹H NMR (400 MHz, DMSO-d6) δ ppm 1.17 (t, 3 H) 1.47 (t, 3 H) 2.67 (s, 3 H) 3.43 (q, 2 H) 4.34 (q, 2H) 5.03 (q, 2 H) 7.37 (d, 1 H) 7.90 (d, 1 H) 11.56 (br s, 1 H) |
| 1.064 | 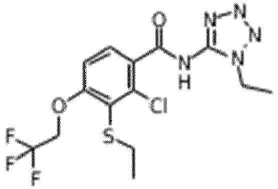 | ¹H NMR (400 MHz, DMSO-d6) δ ppm 1.10 (t, 3 H) 1.47 (t, 3 H) 2.91 (q, 2 H) 4.35 (q, 2 H) 4.99 (q, 2 H) 7.29 (d, 1 H) 7.73 (d, 1 H) 11.60 (br s, 1 H) |
| 1.065 | 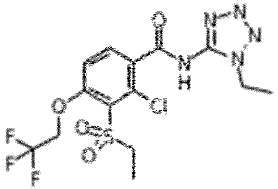 | ¹H NMR (400 MHz, DMSO-d6) δ ppm 1.19 (t, 3 H) 1.47 (t, 3 H) 3.48 (q, 2 H) 4.36 (q, 2 H) 5.08 (q, 2 H) 7.50 (d,1 H) 8.03 (d, 1 H) 11.76 (br s, 1 H) |
| 1.066 | 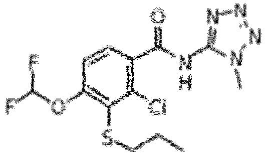 | ¹H NMR (400MHz, CDCI3): 11.06 (s, 1H), 7.70 (d, 1H), 7.29-7.25 (m, 1H), 6.68 (t, 1H), 4.14 (s, 3H), 2.94 (t, 2H), 1.64-1.53 (m, 2H), 1.03 (t, 3H) |
| 1.067 | 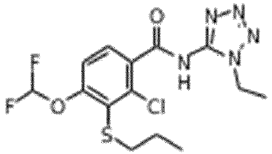 | ¹H NMR (d6-DMSO): 11.84 (1H, s), 8.11 (1H, d), 7.59-7.22 (2H, m), 4.38 (2H, q), 3.53 (2H, t), 1.70 (2H, m), 1.48 (3H, t), 0.99 (3H, t) |
| 1.068 | 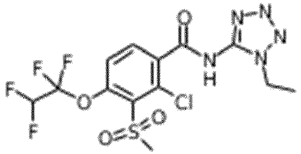 | ¹H NMR (400 MHz, methanol) δ ppm 1.50 - 1.70 (m, 3 H) 3.42 (s, 3 H) 4.39 - 4.51 (m, 2 H) 6.22 - 6.60 (m, 1 H) 7.66 - 7.75 (m, 1 H) 7.97 - 8.06 (m, 1 H) |
| 1.069 | 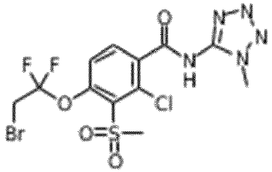 | ¹H NMR (d4 - Methanol): 8.01 (d, 1H), 7.69 (d, 1H), 4.17-4.08 (m, 5H), 3.43(s, 3H) |
| 1.070 | 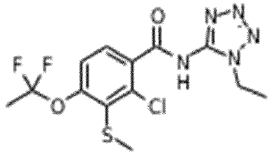 | ¹H NMR (d4 - Methanol): 7.66 (d, 1H), 7.49 (m, 1H), 4.45 (q, 2H), 2.47 (s, 3H), 2.06 (t, 3H), 1.60 (t, 3H) |
| 1.071 | 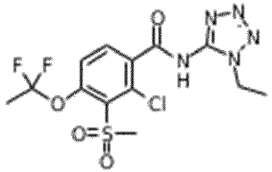 | ¹H NMR (Methanol): 7.94 (d, 1H), 7.65 (d, 1H), 4.43 (m, 2H), 3.38 (s, 3H), 2.05 (m, 3H), 1.58 (m, 3H) |
| 1.072 | 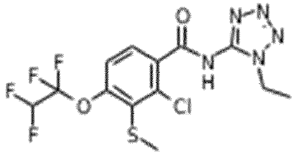 | ¹H NMR (400 MHz, methanol) δ ppm 1.58 (t,3 H) 2.39 - 2.49 (m, 3 H) 4.44 (q, 2 H) 6.30 - 6.64 (m, 1 H) 7.52 (d, 1 H) 7.65 - 7.74 (m, 1 H) |

**TABLE 2 - Examples of herbicidal compounds of the present invention.**

| **COMPOUND** | **STRUCTURE** | **NMR** |
|---|---|---|
| 2.001 | 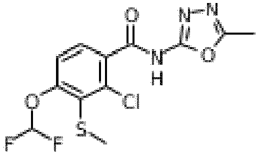 | ¹H NMR(d4-MeOD): 7.67 (d, 1H), 7.19 (d,1H), 6.64 (t,1H), 2.54 (s,3H), 2.46 (s,3H) |
| 2.002 | 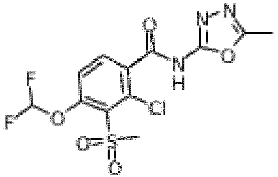 | ¹H NMR(d4-MeOD): 7.87 (d,1H), 7.50 (d,1H), 6.99 (t,1H), 3.39 (s,3H) 2.51(s,3H) |
| 2.003 | 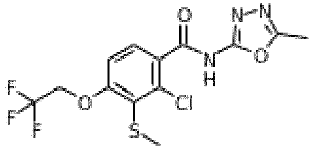 | ¹H NMR(d4-MeOD): 7.54 (d,1H), 7.15 (d, 1H), 4.73 (q,2H), 2.51 (s,3H), 2.42 (s,3H) |
| 2.004 | 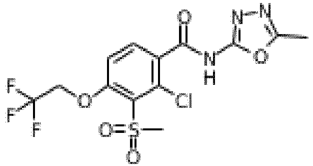 | ¹H NMR(d-MeCN): 7.77 (d,1H), 7.24 (d,1H), 4.73 (q,2H), 3.31 (s,3H), 2.45 (s,3H) |
| 2.005 | 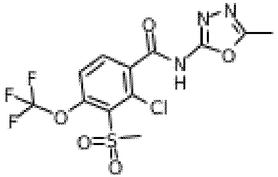 | ¹H NMR(d4-MeOD): 7.98 (d,1H), 7.69 (m,1H), 3.42 (s,3H), 2.54 (s,3H) |
| 2.006 | 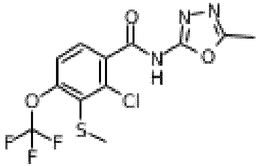 | ¹H NMR(CDCl₃): 7.66 (d,1H), 7.31 (m, 1H), 2.52 (s,3H), 2.46 (s,3H) |
| 2.007 | 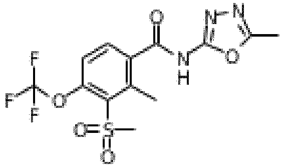 | ¹H NMR (d-MeCN): 7.82 (d,1H), 7.51 (d,1H), 3.29 (s,3H), 2.77 (s,3H), 2.48 (s,3H) |
| 2.008 | 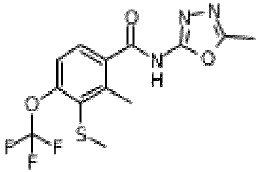 | ¹H NMR (d4-MeOD): 7.57 (d,1H), 7.34 (d,1H), 2.67 (s,3H), 2.51 (s,3H), 2.35 (s,3H) |
| 2.009 | 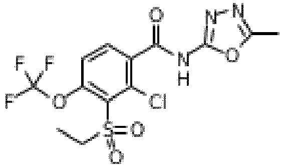 | ¹H NMR (DMSO-d6) δ ppm 12.38- 12.55 (m, 1 H) 8.10 (br d, 1 H) 7.78 (br d,, 1 H) 4.03 (q, 1 H) 3.58 (q, 2 H) 3.40 - 3.48 (m, 1 H) 3.30 (br s, 1 H) 2.57 - 2.60 (m, 1 H) 2.48 - 2.50 (m, 3 H) 2.42 (br s, 1 H) 1.99 (s, 1 H) 1.89 (s, 1 H) 1.34 (s, 1 H) 1.14 - 1.28 (m,1H) |
| 2.010 | 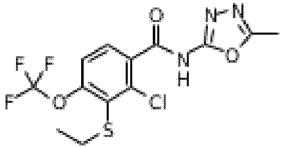 | ¹H NMR (DMSO-d6): 12.35 (br s, 1 H), 7.76 (d, 1 H), 7.65 - 7.55 (m, 1 H), 3.00 - 2.90 (m, 2 H), 2.48 (s, 3 H), 1.14 (t, 3 H) |
| 2.011 | 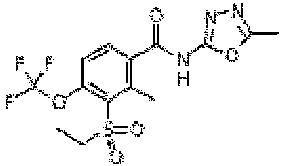 | ¹H NMR (d4-methanol): 7.89 (1H, d), 7.57 (1H, d), 3.50-3.26 (5H, m), 2.79 (3H, s), 2.54 (3H, s), 1.32 (3H, t). |
| 2.012 | 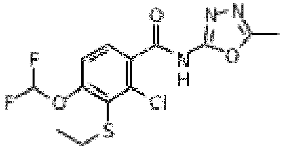 | ¹H NMR (DMSO-d6): 12.23 (br s, 1 H), 7.69 (d, 1 H), 7.42 - 7.24 (m, 2 H), 2.98 - 2.89 (m, 2 H), 2.48 (s, 3 H), 1.13 (t, 3 H) |
| 2.013 | 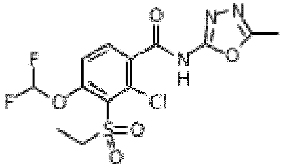 | ¹H NMR (DMSO-d6): 12.39 (br s, 1 H), 8.00 (d, 1 H), 7.59 - 7.17 (m, 2 H), 3.57 - 3.49 (m, 2 H), 2.48 (s, 3 H), 1.22 (t, 3 H) |
| 2.014 | 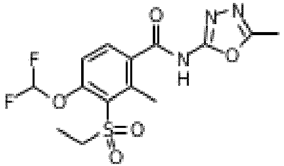 | ¹H NMR (DMSO-d6): 12.19 (br s, 1 H), 7.85 (d, 1 H), 7.52 - 7.16 (m, 2 H), 3.50 - 3.41 (m, 2 H), 2.64 (s, 3 H), 2.49 (s, 3 H), 1.20 (t, 3 H) |
| 2.015 | 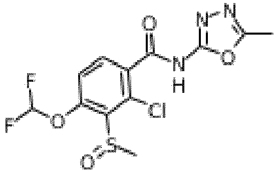 | ¹H NMR(Methanol): 7.83(d, 1H), 7.47(d, 1H), 6.84-7.26(m, 1H), 3.17(s, 3H), 2.51(s, 3H) |
| 2.016 | 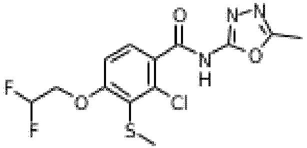 | ¹H NMR (Methanol): 7.52(d, 1H), 7.12(d,1H), 6.12-6.47(m, 1H), 4.40(td, 2H), 2.50(s, 3H), 2.41(s, 3H) |
| 2.017 | 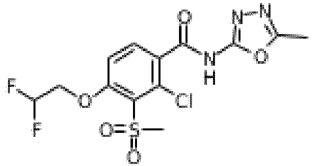 | ¹H NMR(Methanol): 7.78(d, 1H), 7.37 (d, 1H), 6.15-6.53 (m,1H), 4.52(td, 2H), 3.38 (s,3H), 2.50 (s,3H) |
| 2.018 | 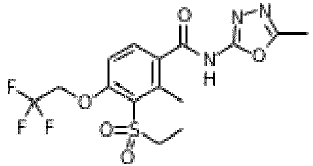 | ¹H NMR (400 MHz, DMSO-d6) δ ppm 1.16 (t, 3 H) 2.47 (s, 3 H) 2.62 (s, 3 H) 3.41 (q, 2 H) 5.00 (q, 2 H) 7.32 (d, 1 H) 7.78 (d, 1 H) 11.97 (br s, 1 H) |
| 2.019 | 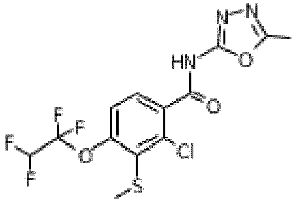 | ¹H NMR (d6-DMSO): 7.76 (1H, d), 7.58 (1H, d), 6.94 (1H, tt), 2.43 (3H, s), 2.21 (3H, s). |

### Biological Examples

Seeds of a variety of test species are sown in standard soil in pots (*Lolium perenne* (LOLPE), *Amaranthus retoflexus* (AMARE), *Abutilon theophrasti* (ABUTH), *Setaria faberi* (SETFA), *Echinochloa crus-galli* (ECHCG), *Ipomoea hederacea* (IPOHE)). After cultivation for one day (pre-emergence) or after 8 days cultivation (post-emergence) under controlled conditions in a glasshouse (at 24/16°C, day/night; 14 hours light; 65 % humidity), the plants are sprayed with an aqueous spray solution derived from the formulation of the technical active ingredient in acetone / water (50:50) solution containing 0.5% Tween^{™} 20 (polyoxyethelyene sorbitan monolaurate, CAS RN 9005-64-5). Compounds are applied at 125 g/h unless otherwise indicated. The test plants are then grown in a glasshouse under controlled conditions in a glasshouse (at 24/16°C, day/night; 14 hours light; 65 % humidity) and watered twice daily. After 13 days for pre- and post-emergence, the test is evaluated for the percentage damage caused to the plant. The biological activities are shown in the following table on a five-point scale (5 = 80-100%; 4 = 60-79%; 3=40-59%; 2=20-39%; 1=0-19%).

**TABLE B1: Pre-Emergence Application**

| **Compound** | **IPOHE** | **ECHCG** | **SETFA** | **ABUTH** | **AMARE** |
|---|---|---|---|---|---|
| 1.003 | 1 | 5 | 5 | 5 | 5 |
| 1 .004* | 1 | 5 | 5 | 5 | 5 |
| 1.005* | 1 | 1 | 1 | 2 | 4 |
| 1.009 | 3 | 5 | 5 | 4 | 5 |
| 1.013 | 5 | 5 | 5 | 5 | 5 |
| 1.014 | 3 | 5 | 5 | 5 | 5 |
| 1.020 | 5 | 5 | 5 | 5 | 5 |
| 1.021 | 3 | 5 | 5 | 4 | 5 |
| 1.022 | 5 | 5 | 5 | 5 | 5 |
| 1.023 | 5 | 5 | 5 | 5 | 5 |
| 1.024 | 5 | 5 | 5 | 5 | 5 |
| 1.025 | 4 | 5 | 5 | 5 | 5 |
| 1.026 | 5 | 5 | 5 | 5 | 5 |
| 1.027 | 3 | 5 | 5 | 5 | 5 |
| 1.028 | 3 | 5 | 5 | 5 | 5 |
| 1.029 | 4 | 5 | 5 | 5 | 5 |
| 1.030 | 3 | 5 | 5 | 5 | 5 |
| 1.031 | 5 | 5 | 5 | 5 | 5 |
| 1.034 | 2 | 4 | 4 | 3 | 4 |
| 1.035 | 5 | 5 | 5 | 5 | 5 |
| 1.036 | 4 | 5 | 5 | 5 | 5 |
| 1.037 | 1 | 4 | 3 | 3 | 5 |
| 1.038 | 2 | 2 | 2 | 4 | 5 |
| 1.039 | 4 | 4 | 4 | 5 | 5 |
| 1.040 | 5 | 5 | 5 | 5 | 5 |
| 1.046 | 5 | 5 | 5 | 5 | 5 |
| 1.047 | 5 | 5 | 5 | 5 | 5 |
| 1.048 | 5 | 5 | 5 | 5 | 5 |
| 1.049 | 5 | 5 | 5 | 5 | 5 |
| 1.050 | 3 | 5 | 5 | 5 | 5 |
| 1.051 | 4 | 5 | 5 | 5 | 5 |
| 1.052 | 5 | 5 | 5 | 5 | 5 |
| 1.053 | 4 | 5 | 5 | 5 | 5 |
| 1.054 | 4 | 5 | 5 | 5 | 5 |
| 1.063 | 4 | 5 | 5 | 5 | 5 |
| 1.064 | 1 | 5 | 2 | 4 | 5 |
| 1.065 | 3 | 5 | 5 | 5 | 5 |
| 1.066 | 3 | 5 | 5 | 5 | 5 |
| 1.067 | 2 | 5 | 4 | 4 | 5 |
| 2.005* | 1 | 5 | 5 | 5 | 5 |
| 2.006* | 1 | 1 | 1 | 2 | 5 |
| 2.009 | 2 | 5 | 5 | 5 | 5 |
| 2.010 | 1 | 5 | 5 | 4 | 5 |
| 2.011 | 5 | 5 | 5 | 5 | 5 |
| 2.012 | 3 | 5 | 5 | 4 | 5 |
| 2.013 | 4 | 5 | 5 | 5 | 5 |
| 2.014 | 5 | 5 | 5 | 5 | 5 |
| 2.018 | 4 | 5 | 5 | 5 | 5 |

**TABLE B2: Post-Emergence Application**

| **Compound** | **IPOHE** | **ECHCG** | **SETFA** | **ABUTH** | **AMARE** |
|---|---|---|---|---|---|
| 1.003 | 5 | 5 | 5 | 5 | 5 |
| 1 .004* | 5 | 5 | 5 | 5 | 5 |
| 1.005* | 5 | 5 | 5 | 5 | 5 |
| 1.009 | 4 | 4 | 4 | 4 | 4 |
| 1.013 | 5 | 5 | 5 | 5 | 5 |
| 1.014 | 5 | 5 | 5 | 5 | 5 |
| 1.020 | 5 | 5 | 5 | 5 | 5 |
| 1.021 | 5 | 5 | 5 | 5 | 4 |
| 1.022 | 5 | 5 | 5 | 5 | 4 |
| 1.023 | 5 | 5 | 5 | 5 | 5 |
| 1.024 | 5 | 5 | 5 | 5 | 5 |
| 1.025 | 5 | 5 | 5 | 5 | 5 |
| 1.026 | 5 | 5 | 5 | 5 | 5 |
| 1.027 | 4 | 5 | 5 | 5 | 5 |
| 1.028 | 5 | 5 | 5 | 5 | 5 |
| 1.029 | 5 | 5 | 5 | 5 | 5 |
| 1.030 | 5 | 5 | 5 | 5 | 5 |
| 1.031 | 5 | 5 | 5 | 5 | 5 |
| 1.034 | 3 | 5 | 4 | 4 | 4 |
| 1.035 | 5 | 5 | 5 | 5 | 5 |
| 1.036 | 5 | 5 | 5 | 5 | 5 |
| 1.037 | 3 | 5 | 5 | 5 | 5 |
| 1.038 | 4 | 5 | 5 | 5 | 5 |
| 1.039 | 4 | 5 | 5 | 5 | 5 |
| 1.040 | 5 | 5 | 5 | 5 | 5 |
| 1.046 | 5 | 5 | 5 | 5 | 5 |
| 1.047 | 5 | 5 | 5 | 5 | 5 |
| 1.048 | 5 | 5 | 5 | 5 | 5 |
| 1.049 | 4 | 5 | 5 | 5 | 5 |
| 1.050 | 4 | 5 | 5 | 5 | 5 |
| 1.051 | 4 | 5 | 5 | 5 | 5 |
| 1.052 | 5 | 4 | 4 | 5 | 4 |
| 1.053 | 5 | 5 | 5 | 5 | 5 |
| 1.054 | 4 | 4 | 4 | 4 | 4 |
| 1.063 | 3 | 5 | 5 | 5 | 5 |
| 1.064 | 4 | 5 | 5 | 5 | 5 |
| 1.065 | 4 | 5 | 5 | 5 | 5 |
| 1.066 | 5 | 4 | 4 | 5 | 5 |
| 1.067 | 4 | 4 | 4 | 5 | 5 |
| 2.005* | 5 | 5 | 5 | 5 | 5 |
| 2.006* | 5 | 5 | 5 | 5 | 5 |
| 2.009 | 4 | 5 | 5 | 4 | 5 |
| 2.010 | 4 | 5 | 5 | 5 | 5 |
| 2.011 | 4 | 5 | 5 | 5 | 5 |
| 2.012 | 4 | 5 | 5 | 5 | 5 |
| 2.013 | 4 | 5 | 5 | 5 | 5 |
| 2.014 | 4 | 5 | 5 | 5 | 5 |
| 2.018 | 5 | 5 | 5 | 5 | 5 |

| | | | | | |
|---|---|---|---|---|---|
| *Applied at 250g/ha | | | | | |

A comparative experiment is conducted to show the advantage provided by the compounds of the present invention. Thus the biological performance of representative compounds 1.004 and with Compound 4-460 of the type referred to in WO2012/028579. Results are given as (%) phytotoxicity observed. The result demonstrates that compounds of the present invention exhibit much improved crop (ZEAMX / maize) selectivity - that is they provide improved control of problematic weed species, whilst exhibiting little if any crop damage at like-for-like application rates.

**TABLE B3: Comparative Experiment**

| Compound | Rate g/ha | POST Application | | | | | | |
|---|---|---|---|---|---|---|---|---|
| | | **ZEAMX** | IPOHE | ECHCG | SETFA | ABUTH | DIGSA | AMARE |
| 1.003 | 30 | 0 | 100 | 90 | 90 | 100 | 80 | 100 |
| 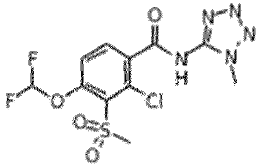 | | | | | | | | |
| | 15 | 0 | 100 | 90 | 80 | 90 | 80 | 100 |
| 1.004 | 30 | 30 | 100 | 100 | 100 | 100 | 100 | 100 |
| 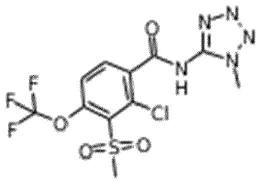 | | | | | | | | |
| | 15 | 0 | 90 | 90 | 100 | 90 | 80 | 100 |
| Compound 4-640 | 30 | 70 | 90 | 90 | 90 | 90 | 80 | 90 |
| WO2012/028579 | | | | | | | | |
| 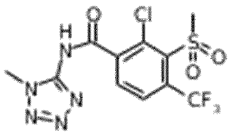 | | | | | | | | |
| | 15 | 40 | 90 | 80 | 90 | 90 | 80 | 90 |

## Claims

1. A compound of Formula (I): 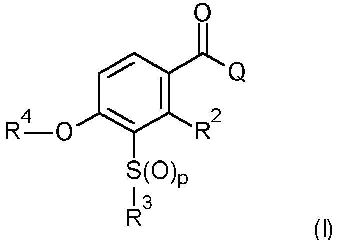 or an agronomically acceptable salt thereof,
wherein
Q is selected from the group consisting of Q1 and Q2: 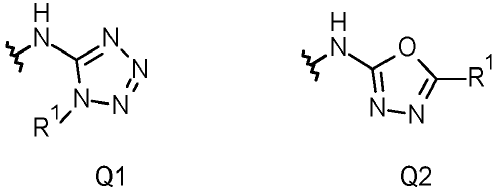
R¹ is selected from the group consisting of C₁-C₄alkyl-, C₁-C₄haloalkyl-, C₁-C₄alkoxy-C₁-C₄alkyl- and C₁-C₄haloalkoxy-C₁-C₄alkyl-;
R² is selected from the group consisting of halogen, C₁-C₆alkyl-, C₁-C₃alkoxy-, C₁-C₆ haloalkyl-, C₁-C₃haloalkoxy- and -S(O)ₚC₁-C₆alkyl;
R³ is selected from the group consisting of C₁-C₆alkyl-, C₃-C₆cycloalkyl- and C₁-C₆ haloalkyl-;
R⁴ is C₁-C₆haloalkyl;
and
p is 0, 1 or 2.

2. A compound according to claim 1, wherein Q is Q1.

3. A compound according to claim 1, wherein Q is Q2.

4. A compound according to any one of the previous claims, wherein R¹ is C₁-C₄alkyl-.

5. A compound according to claim 4, wherein R¹ is methyl.

6. A compound according to any one of the previous claims, wherein R² is selected from the group consisting of chlorine, methyl and CF₃.

7. A compound according to claim 6, wherein R² is chlorine.

8. A compound according to any one of the previous claims, wherein R³ is C₁-C₆alkyl-.

9. A compound according to claim 8, wherein R³ is methyl or ethyl.

10. A compound according to any one of the previous claims, wherein R⁴ is CF₃ or CHF₂.

11. A herbicidal composition comprising a compound according to any one of the previous claims and an agriculturally acceptable formulation adjuvant.

12. A herbicidal composition according to claim 11, further comprising at least one additional pesticide.

13. A method of controlling weeds at a locus comprising application to the locus of a weed controlling amount of a composition according to any one of claims 11 to 12.

14. Use of a compound of Formula (I) as defined in claim 1 as a herbicide.

15. A compound of Formula (Va) 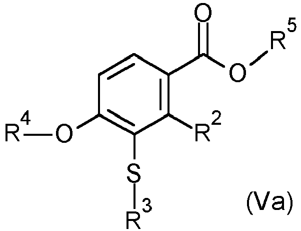 wherein R², R³ and R⁴ are as defined in claim 1 above and R⁵ is hydrogen or C₁-C₄ alkyl.

## Patentansprüche

1. Verbindung der Formel (I): 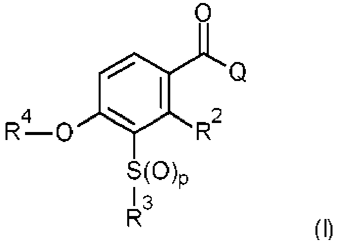 oder ein agronomisch unbedenkliches Salz davon,
wobei:
Q aus der aus Q1 und Q2 bestehenden Gruppe ausgewählt ist: 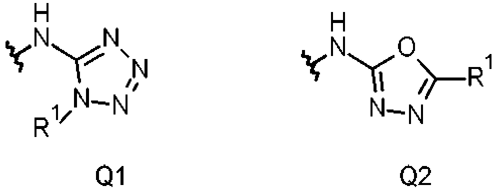
R¹ aus der aus C₁-C₄-Alkyl-, C₁-C₄-Halogenalkyl-, C₁-C₄-Alkoxy-C₁-C₄-alkyl- und C₁-C₄-Halogenalkoxy-C₁-C₄-alkyl-bestehenden Gruppe ausgewählt ist,
R² aus der aus Halogen, C₁-C₆-Alkyl-, C₁-C₃-Alkoxy-, C₁-C₆-Halogenalkyl-, C₁-C₃-Halogenalkoxy- und -S(O)ₚ-C₁-C₆-Alkyl bestehenden Gruppe ausgewählt ist,
R³ aus der aus C₁-C₆-Alkyl-, C₃-C₆-Cycloalkyl- und C₁-C₆-Halogenalkyl- bestehenden Gruppe ausgewählt ist,
R⁴ für C₁-C₆-Halogenalkyl steht
und
p für 0, 1 oder 2 steht.

2. Verbindung nach Anspruch 1, wobei Q für Q1 steht.

3. Verbindung nach Anspruch 1, wobei Q für Q2 steht.

4. Verbindung nach einem der vorhergehenden Ansprüche, wobei R¹ für C₁-C₄-Alkyl- steht.

5. Verbindung nach Anspruch 4, wobei R¹ für Methyl steht.

6. Verbindung nach einem der vorhergehenden Ansprüche, wobei R² aus der aus Chlor, Methyl und CF₃ bestehenden Gruppe ausgewählt ist.

7. Verbindung nach Anspruch 6, wobei R² für Chlor steht.

8. Verbindung nach einem der vorhergehenden Ansprüche, wobei R³ für C₁-C₆-Alkyl- steht.

9. Verbindung nach Anspruch 8, wobei R³ für Methyl oder Ethyl steht.

10. Verbindung nach einem der vorhergehenden Ansprüche, wobei R⁴ für CF₃ oder CHF₂ steht.

11. Herbizide Zusammensetzung, umfassend eine Verbindung nach einem der vorhergehenden Ansprüche und ein landwirtschaftlich unbedenkliches Formulierungshilfsmittel.

12. Herbizide Zusammensetzung nach Anspruch 11, ferner umfassend mindestens ein zusätzliches Pestizid.

13. Verfahren zur Bekämpfung von Unkräutern an einem Standort, bei dem man eine unkrautbekämpfende Menge einer Zusammensetzung nach einem der Ansprüche 11 bis 12 auf den Standort ausbringt.

14. Verwendung einer Verbindung der Formel (I) gemäß Anspruch 1 als ein Herbizid.

15. Verbindung der Formel (Va) 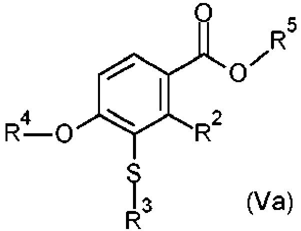 wobei R², R³ und R⁴ jeweils wie oben in Anspruch 1 definiert sind und R⁵ für Wasserstoff oder C₁-C₄-Alkyl steht.

## Revendications

1. Composé de Formule (I) : 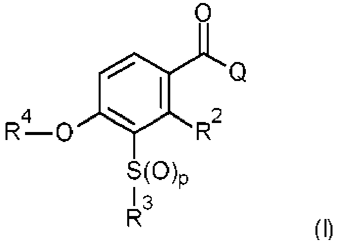 ou sel acceptable sur le plan agronomique de celui-ci,
Q étant choisi dans le groupe constitué par Q1 et Q2 : 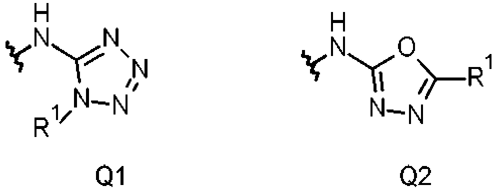
R¹ étant choisi dans le groupe constitué par C₁-C₄alkyle-, C₁-C₄halogénoalkyle-, C₁-C₄alcoxy- C₁-C₄alkyle- et C₁-C₄halogénoalcoxy-C₁-C₄alkyle- ;
R² étant choisi dans le groupe constitué par halogène, C₁-C₆alkyle-, C₁-C₃alcoxy-, C₁-C₆ halogénoalkyle-, C₁-C₃halogénoalcoxy- et -S(O)ₚC₁-C₆alkyle ;
R³ étant choisi dans le groupe constitué par C₁-C₆alkyle-, C₃-C₆cycloalkyl- et C₁-C₆ halogénoalkyle- ;
R⁴ étant C₁-C₆halogénoalkyle ;
et
p étant 0, 1 ou 2.

2. Composé selon la revendication 1, Q étant Q1.

3. Composé selon la revendication 1, Q étant Q2.

4. Composé selon l'une quelconque des revendications précédentes, R¹ étant C₁-C₄alkyle-.

5. Composé selon la revendication 4, R¹ étant méthyle.

6. Composé selon l'une quelconque des revendications précédentes, R² étant choisi dans le groupe constitué par chlore, méthyle et CF₃.

7. Composé selon la revendication 6, R² étant chlore.

8. Composé selon l'une quelconque des revendications précédentes, R³ étant C₁-C₆alkyle-.

9. Composé selon la revendication 8, R³ étant méthyle ou éthyle.

10. Composé selon l'une quelconque des revendications précédentes, R⁴ étant CF₃ ouCHF₂.

11. Composition herbicide comprenant un composé selon l'une quelconque des revendications précédentes et un adjuvant de formulation acceptable sur le plan agricole.

12. Composition herbicide selon la revendication 11, comprenant en outre au moins un pesticide supplémentaire.

13. Procédé de lutte contre des mauvaises herbes au niveau d'un lieu, comprenant une application sur le lieu d'une quantité de lutte contre des mauvaises herbes d'une composition selon l'une quelconque des revendications 11 à 12.

14. Utilisation d'un composé de Formule (I) tel que défini selon la revendication 1, comme herbicide.

15. Composé de formule (Va) 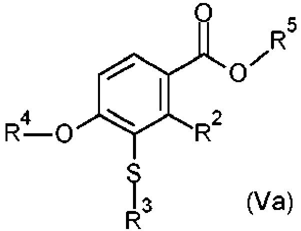 R², R³ et R⁴ étant tels que définis dans la revendication 1 ci-dessus et R⁵ étant hydrogène ou C₁-C₄ alkyle.
